# EUROPEAN PATENT SPECIFICATION

(11) **EP 2 904 103 B1**
(45) Date of publication and mention of the grant of the patent: **22.01.2020**
(21) Application number: 13844080.5
(22) Date of filing: 30.09.2013
(51) Int. Cl.: C12N 15/867, C12N 5/10, A61P 1/16, A61P 11/00, A61K 38/45, A61K 38/46, A61K 35/545

(54) **METHOD FOR PREPARING INDUCED PLURIPOTENT STEM CELLS AND ITS APPLICATIONS**
VERFAHREN ZUR HERSTELLUNG INDUZIERTER PLURIPOTENTER STAMMZELLEN UND DEREN ANWENDUNGEN
PROCÉDÉ DE PRÉPARATION DE CELLULES SOUCHES PLURIPOTENTES INDUITES ET SES APPLICATIONS

(30) Priority: 01.10.2012 US 201261708128 P; 24.10.2012 US 201261717871 P
(43) Date of publication of application: 12.08.2015
(73) Proprietor: Taipei Veterans General Hospital, Taipei City, Taiwan 11217 (TW)
(72) Inventor: CHIEN, Yueh, Taipei City 11217 Taiwan (TW); CHIOU, Guang-Yuh, Taipei City 11217 Taiwan (TW); YANG, Yi-Ping, Taipei City 11267 (TW)
(74) Representative: Hoefer & Partner Patentanwälte mbB
(86) International application number: PCT/CN2013/001187
(87) International publication number: WO 2014/053082

(56) References cited:
- WO-A1-2012/116111
- CN-A- 102 653 774
- US-B1- 8 048 675
- US-B2- 8 058 065
- MASSIMO DE FELICI: "Nuclear Reprogramming in Mouse Primordial Germ Cells: Epigenetic Contribution", STEM CELLS INTERNATIONAL, vol. 122, no. 4, 1 January 2011 (2011-01-01), pages 1235-15, XP055249427, DOI: 10.1016/j.modgep.2004.12.007
- LOH KYLE M ET AL: "Epigenetics: Actors in the cell reprogramming drama", NATURE, NATURE PUBLISHING GROUP, UNITED KINGDOM, vol. 488, no. 7413, 30 August 2012 (2012-08-30), pages 599-600, XP009188497, ISSN: 1476-4687
- OEGE, CLAUDIA A. ET AL.: 'Early-stage epigenetic modification during somatic cell eprogramming by Parpl and Tet2.' NATURE. vol. 488, 19 August 2012, pages 652 - 655, XP055248925
- LI, HSIN-YANG ET AL.: 'Reprogramming induced pluripotent stem cells in the absence of c-Myc for differentiation into hepatocyte-like cells.' BIOMATERIALS vol. 32, 11 June 2011, pages 5994 - 6005, XP028097254
- LAI, YI-SHIN ET AL.: 'SRY (sex determining region Y)-box2 (Sox2)/poly ADP-ribose polymerase 1 (Parpl) complexes regulate pluripotency.' PNAS. vol. 109, no. 10, 06 March 2012, pages 3772 - 3777, XP055248928
- CHIOU, SHIH-HWA ET AL.: 'Poly(ADP-ribose) polymerase 1 regulates nuclear reprogramming and promotes iPSC generation without c-Myc.' JEM. vol. 210, no. 1, 31 December 2012, pages 85 - 98, XP055194521

## Description

### CROSS REFERENCE TO RELATED APPLICATIONS

This patent application claims priority to U.S. provisional patent application Ser. No. 61/708,128filed on October 1, 2012. This application also claims priority to U.S. provisional patent application Ser. No. 61/717,871filed on October24, 2012.

### FIELD OF THE INVENTION

The present invention relates generally to a method for preparing induced pluripotent stem cells.

### BACKGROUND OF THE INVENTION

The cellular reprogramming technology to generate induced pluripotent stem (iPSCs) is a promising strategy for stem cell biology and regenerative medicine. Accumulated data have shown that nuclear reprogramming can be experimentally induced by three methods: nuclear transfer, cell fusion, or forced expression of transcription factors (Yamanaka and Blau, 2010).It is conceivable that mature oocytes and embryonic stem cells (ESCs) contain reprogramming factors (proteins, RNAs, lipids, and small molecules) that enable these somatic cells to undergo efficient nuclear reprogramming, a process converting somatic cells to pluripotent states (Jullien *et al*., 2010; Wang *et al*., 2010). Recent evidence has emphasized the pivotal roles of nuclear proteins in the regulation of chromatin remodeling and epigenetic modifications during the reprogramming process(Jullien *et al*., 2011). However, the precise molecular mechanisms of the regulation of nuclear factors during cellular reprogramming remain uncertain.

Induced pluripotent stem cells (iPSCs) were first generated by Shinya Yamanaka's team, who was awarded the Nobel Prize in medicine in 2012.Yamanaka successfully produced iPSCs by the transfection of the genes that had been identified as particularly important in embryonic stem cells (ESCs), and isolated four key pluripotency genes essential for the production of pluripotentstem cells: Oct-3/4, Sox2, c-Myc, and Klf4 (Takahashi and Yamanaka, 2006). Then, it is reported that nuclear reprogramming induced by transcription factors resets the epigenetic landmarks, leading to the global reversion of the somatic epigenomes to an ESC-like state (Maherali *et al*., 2007; Papp and Plath, 2011). However, the mechanisms involved in the posttranslational interaction and modification remain undetermined. Mass spectrometry (MS)-based proteomics is the most powerful tool available for global investigation of proteome profiles in stem cell biology (Rigbolt *et al*., 2011 ; Van Hoof *et al*., 2009). Although the importance of nuclear proteins in epigenetic events has been addressed (Jullien *et al*., 2010), little was known about the involving functional proteins and mechanisms that regulate reprogramming and maintain pluripotency.

Yamanaka *et al.* also provided the method for preparing an induced pluripotent stem cell (iPSC)by nuclear reprogramming of a somatic cell through introducing the genes Oct3/4, Klf4, c-Myc and Sox2in US Patent No. 8,058,065 (filed on June 9, 2009 and issued on November 15, 2011). The method for preparing somatic cells by inducing differentiation of the iPSC as obtained was disclosed in US Patent No. 8,129,187 (filed on February 18, 2010 and issued on March 6, 2012). Then, Yamanaka *et al.* provided a method for preparing an induced pluripotent stem cell (iPSC) by introducing the genes encoding Oct3/4, Klf4 and Sox2without c-Myc (US Patent No. 8,278,104, filed on June 13, 2008 and issued on October 2, 2012). Sakurada *et al.* provides a method and platform for drug discovery using two or more populations of isolated cells differentiated from the iPSCs comprising an exogenous Oct3/4, Sox2 and Klf4 genes (US Patent No. 8,257,941, filed on June 12, 2009 and issued on September 4, 2012).

Irion provided a method for generating integration-free human induced pluripotent stem cells from blood cells using one or more DNA expression vectors encoding the reprogramming factors (a) Oct4, Sox2, Klf4, and c-Myc; (b) Oct4, Sox2, and Klf4; (c) Oct4, Sox2, Klf4, c-Myc, and Nanog; or (d) Oct 4, Sox2, Lin-28, and Nanog (US Patent No. 8,048,675, filed on May 12, 2010 and issued on November 1, 2011).

Therefore, it is important to identify novel nuclear factors involved in the regulation of nuclear reprogramming using a proteomic approach, in order to elucidate the complex molecular networks in the nucleus during the reprogramming process.

Induced pluripotent stem cells (iPSCs) can be reprogrammed from adult somatic cells by the transduction of genes or chemical agents. iPSCs share the features of embryonic stem cells (ESCs) and are capable of self-renewal and tridermal differentiation, offering a resource for disease modeling and a potentially source for transplantation. The major advantage of iPS cells over embryonic stem (ES) cells is that iPS cells can be derived from a patient's own somatic cells, thereby avoiding immune rejection after transplantation and the ethical concerns raised by using ES cells. Recently, human cystic fibrosis iPSCs were demonstrated to produce disease-specific lung progenitor cells and eventually form respiratory epithelium in immunodeficient mice (Mou H, Zhao R, et al. Cell Stem Cell 2012;10:385-397). In addition, human iPSCs are capable of forming myogenic progenitors and neurons, leading to functional recovery after the transplantation into neuromuscular disorder or stroke disease models (Darabi R et al. Cell Stem Cell 2012;10:610-619; and Oki K et al. Stem Cells 2012;30:1120-1133). However, the possible protective role of iPSCs and the underlying mechanisms remain unknown.

### BRIEF SUMMARY OF THE INVENTION

It is unexpected found in the present invention that induced pluripotent stem cells (iPSCs) could be successfully prepared by transfecting somatic cells to expressOct3/4, Sox2, and Parp1, without c-Myc and/or Klf4 that are oncogenic.In the present description it is also disclosed that interferon (IFN)-γ inducible protein-10 (IP-10), a chemokine responsible for repairing or remodelling a tissue injury repair, was involved in the effect mediated by pluripotent stem cells (iPSCs) or iPSC-conditioned medium (iPSC-CM).

Accordingly, in one aspect, the present invention provides a method as defined in the claims for preparinginduced pluripotent stem cells (iPSCs) from somatic cells without using c-Myc and/or Klf4. The method comprises (a) transfecting isolated somatic cells to express Oct3/4, Sox2 and Parp1; and (b) culturing the transfected somatic cells as obtained in step (a) under appropriate conditions, thereby converting the somatic cells into iPSCs and maintaining pluripotency and self-renewal ability.

In another aspect,the present invention provides a method for preparing iPSCs from somatic cells, comprising: (a) contacting or exposing isolated somatic cells with/to Oct3/4, Sox2 and Parp1, and (b) culturing the somatic cells as obtained in step (a) under appropriate conditions, thereby converting, at least a subset of, the population of somatic cells into iPSCs and maintaining pluripotency and self-renewal ability.

The present description also discloses a method of reprogramming adult cells comprising administering the cells with a protein that is/can be PARylated, or an enzyme that has PARylation activity.

The present description also discloses a method of rejuvenating cells or senescent cells in a subject, comprising administering to the subject a protein that is able to be PARylated, or an enzyme that has PARylation activity.

The present description also discloses a method for inducing the secretion of IP-10 which comprisesadministering to a subject in need thereof an effective amount of iPSCs or iPSC-CM.

The present description also discloses a method for treatingtissue injuries which comprises administering to a subject in need thereof a therapeutically effective amount of iPSCs or iPSC-CM, wherein the therapeutically effective amount is an amount capable of inducing a sufficient level of IP-10 tosuppressinflammation responses.

The present description also discloses a composition comprising iPSCs or iPSC-CM for inducing the secretion of IP-10 in a subject.

It is to be understood that both the foregoing general description and the following detailed description are exemplary and explanatory only and are not restrictive of the invention.

### BRIEF DESCRIPTION OF THE SEVERAL VIEWS OF THE DRAWINGS

The foregoing summary, as well as the following detailed description of the invention, will be better understood when read in conjunction with the appended drawings (*).

In the drawings: *: Drawings relating to subject-matter not encompassed by the claims are for reference.

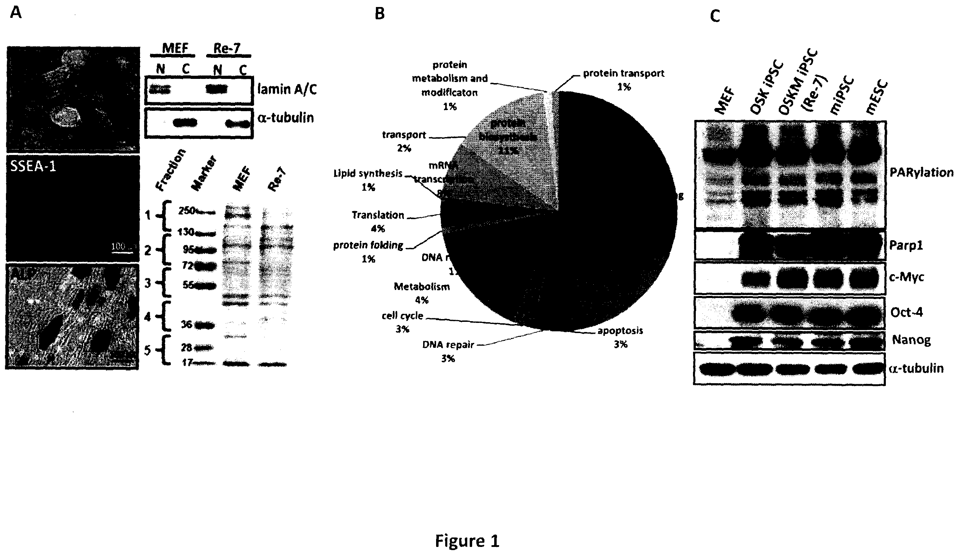

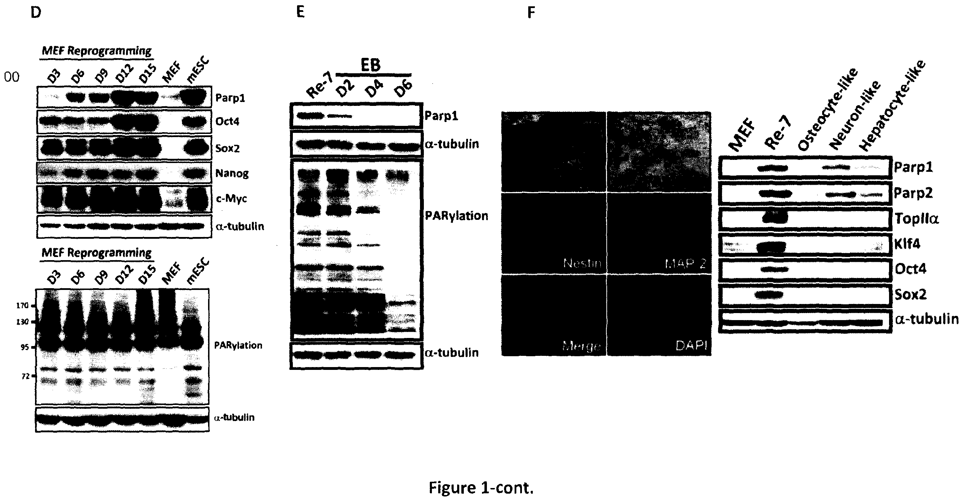

Figure 1 shows Parp1 and PARylation activity in the pluripotent or differentiated state. Panel A: Morphology and staining for ALP and SSEA-1 in Re-7 iPSCs. BF, bright field. Bars=100 µm. Nuclear proteins from Re-7 iPSCs and MEFs were separated into five fractions by 1D-DIGE. Panel B: Pie chart showing the GO classification of gene functions in all nuclear proteins from iPSCs. Panel C: Expression of Oct4, Nanog, c-Myc, Parp1 and PARylation activity in pluripotent stem cells, including ESCs and iPSCs transfected with OSKM or OSK.Panel D: *Upper*:Expansion of the pluripotency factors Oct4, Sox2, Klf4, c-Myc and Parp1;*Lower*: PARylation activity during the reprogramming process. Panel E: Parp1 expression and PARylation activity in EBs 6 days after differentiation. Panel F:*Left:* Differentiation of Re-7 iPSCs into osteocyte-like, hepatocyte-like or neuron-like cells,confirmed by positive staining with Alizarin red, PAS, and the neuron-specific markers Nestin and MAP2, respectively. Bars=100 µm. *Right:* Detection of Parp1, Parp2, TopII-alpha, Oct4, Sox2, and K1f4 proteins in Re-7 iPSCs, before and after differentiation into the indicated specific lineages.The western blots are representative of three separate experiments with independent cell preparations.

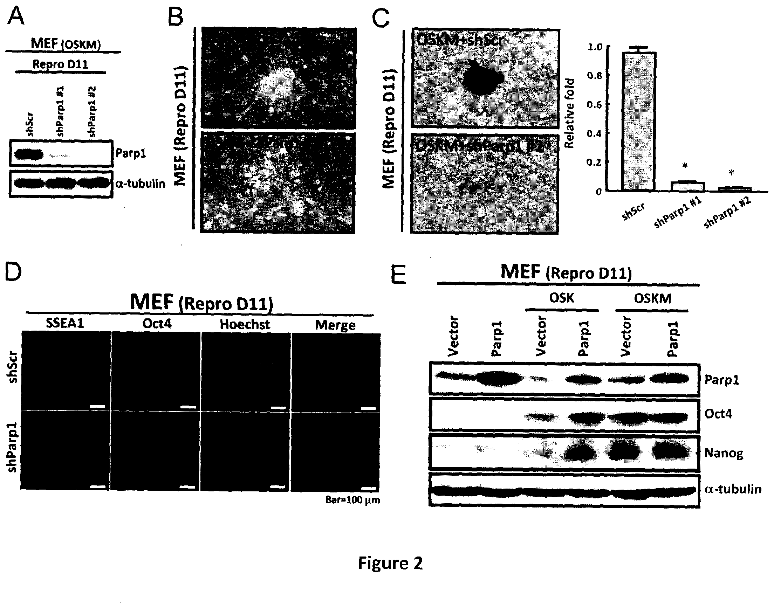

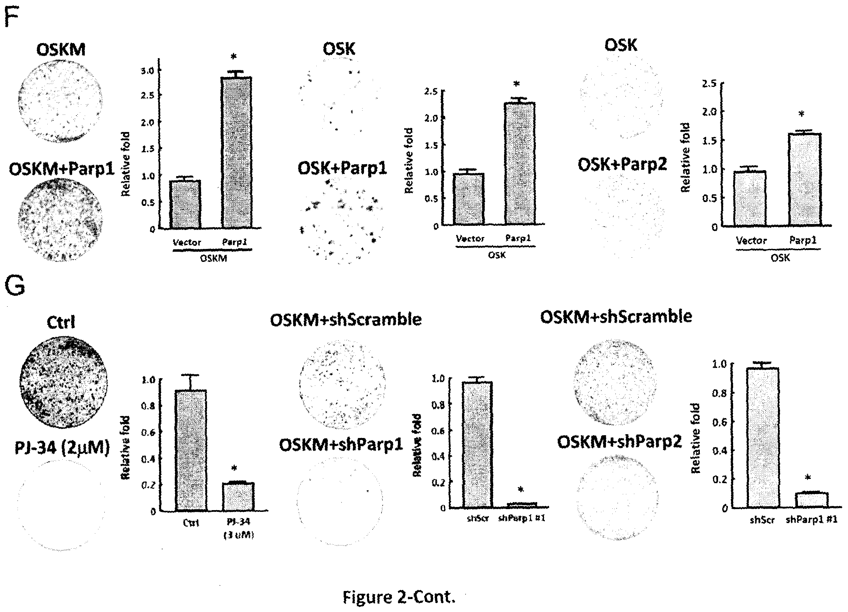

Figure 2 shows that Parp1 expression and PARylation activity are essential for iPSC reprogramming. Panel A:Western blotting confirms the effect of Parp1 knockdown on Parp1 expression at reprogramming day 11 (Repro D11).Panel B: Parp1 knockdownin MEFs transfected with OSKM affects self-renewal and proliferative capabilitiesduring the reprogramming process. Panel C: Reprogramming cells stained with ALP demonstrate the colony size of iPSCs with Parp1 knockdown.Pane1 D:Immunofluorescent staining for ESC markers SSEA-1 and Oct4 in iPSCs with Parp1 knockdown at Repro D11.Panel E:Detection of Parp1 and Oct4 expression in OSK- or OSKM-transfected MEFs overexpressing Parp1 at Repro D11. Panel F:Reprogramming cells stained with ALP and the relative reprogramming efficiencies of MEFs transfected with OSKMwith or without Parp1 overexpression (*Left*)*,* OSK with or without Parp1 overexpression (*Middle*)*,* and OSK with or without Parp2 overexpression (*Right*)*,* at day 21 post-reprogramming.Panel G: *Left:* Reprogramming cells stained with ALP and reprogramming efficiencies of reprogramming cells (OSKM) treated with the PARylation inhibitor PJ-34, compared to cells without the inhibitor. OSKM-transfected MEFs treated with ShRNA-Parp1(*Middle*)or ashRNA-scrambled control(Right), at day 21 post-reprogramming. The western blots are representative of three separate experiments with independent cell preparations. The ALP staining shown here are the mean ±SD of six independent experiments. *P< 0.05 vs. Parental.

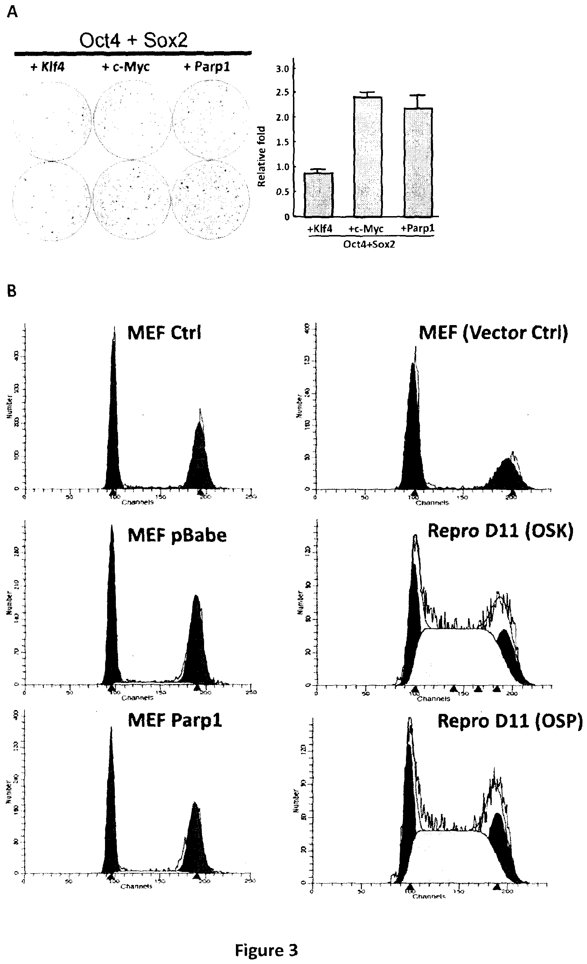

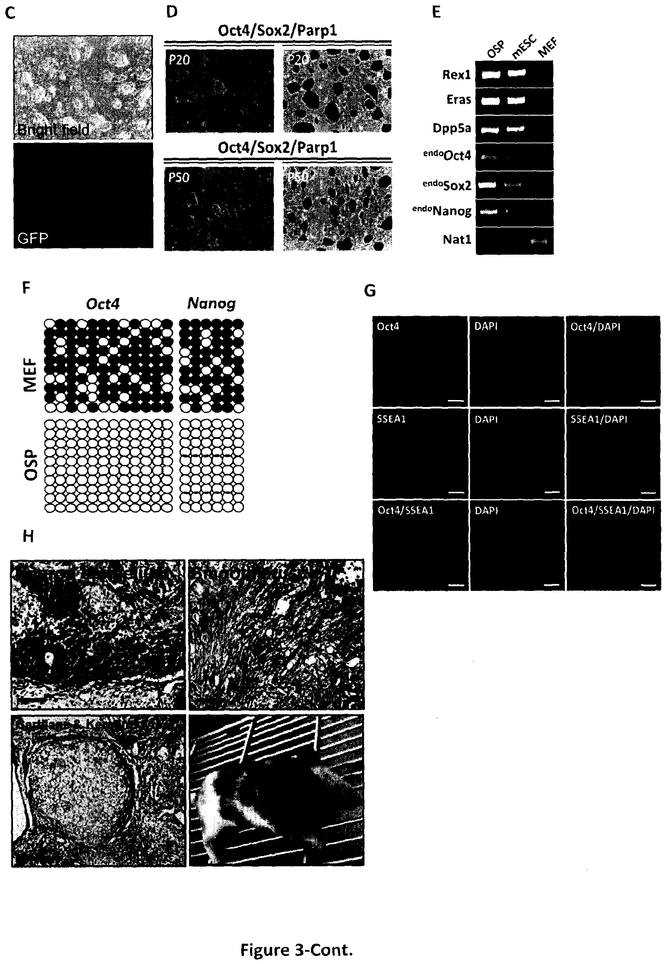

Figure 3 shows that Parp1 is able to replace K1f4 or c-Myc to generate chimeric mice. Panel A: Comparison of reprogramming efficiencies between OSK, OSM, and OSP by ALP-positive colony counting. Panel B:Flow cytometry showing the cell cycle analysis in MEFs transfected with control vector or Parp1 at reprogramming day 0 (*left*) or day 11 (*right*)*.* Panel C: Activation of the expression of Nanog-GFP by OSP transfection during reprogramming in a Nanog-GFP reporter MEF clone. Panel D:OSP-generated iPSCs can be stably cultured to at least 50 passages with high ALP activity.Panel E: RT-PCR showing the ESC-like gene signature in OSP-generated iPSCs at the 50^{th} passage. Panel F: Immunofluorescence indicating the protein expression of pluripotency factors in OSP- iPSCs at the 50^{th} passage.Panel G:Bisulfite sequencing showing the methylation profiles of the promoters of Oct4 and Nanog in MEFs and OSP-iPSCs. Open and filled circles indicate unmethylated and methylated CpG dinucleotides, respectively.Panel H*:Ex vivo* biopsies and histological analysis reveal teratoma formation in the subrenal grafts of OSP-iPSCs in nude mice. Competence of OSP-iPSCs to generate chimeric mice, as confirmed by coat color (*lower right*)*.* Bars=100 µm. The ALP staining shown here are the mean ±SD of six independent experiments.The western blots and other data are representative of three separate experiments with independent cell preparations.

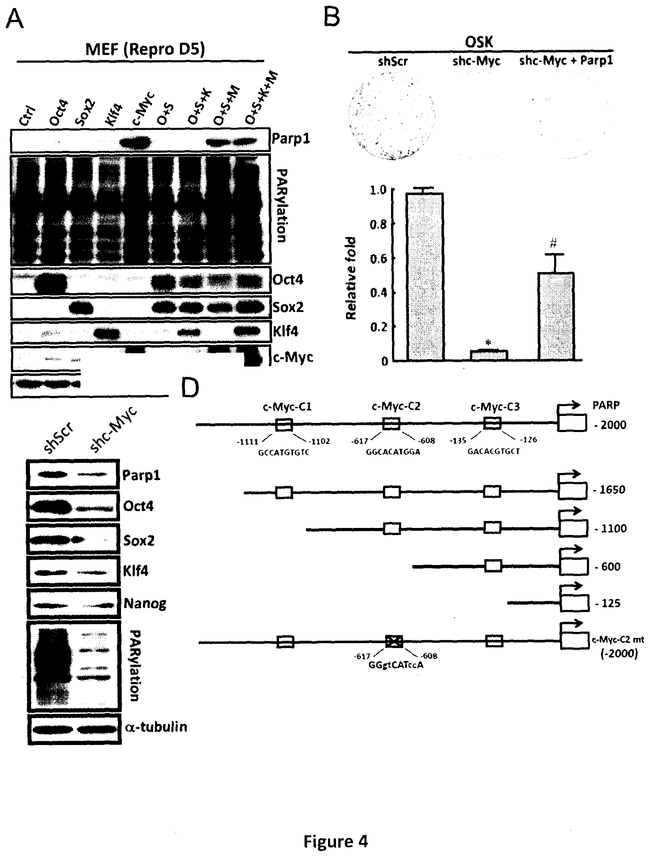

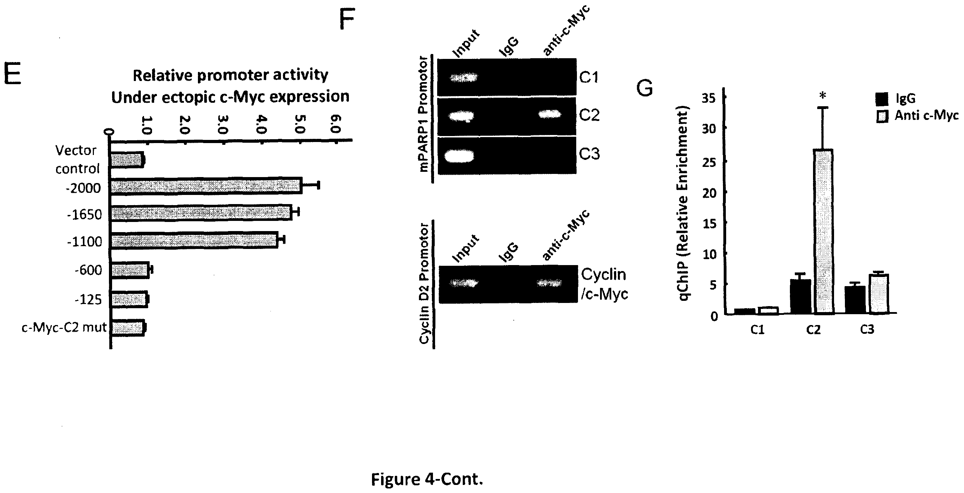

Figure 4 showsthatc-Myc is a key factor regulating Parp1 expression and PARylation activity. Panel A: Western blots showing Parp1 expression and PARylation level at Day 5 post MEF cell transfection with indicated factors. Panel B: Reprogramming cells stained with ALP showing relative reprogramming efficiencies of cells expressing OSK and scrambled shRNA, OSK and c-Myc shRNA, or OSK, c-Myc shRNA and Parp1.Panel C: Expression of Parp1, Oct4, Sox2, Klf4, and Nanog as well as PARylation activity in pluripotent stem cells treated with c-Myc shRNA.Panel D:Schematic representation of the reporter constructs containing serially deleted or point mutated mouse Parp1 promoter. Panel E: Luciferase activities of reporter constructs containing serially deleted or point mutated mouse Parp1 promoter as indicated in panel D.Panel F:Chromatin immunoprecipitation (ChIP) analysis with IgG or c-Myc Ab for immunoprecipitation, followed by PCR using C1, C2, and C3 primer sets for Parp1 promoter and primers for cyclin D2 promoter.Panel G: The quantitative result of panel F (ChIP) with qPCR (qChIP). Input, 2% of total lysate. The western blots are representative of three separate experiments with independent cell preparations. The ALP staining(panel B) shown here are the mean ±SD from six independent experiments and other experiments (panels E and G) are from three independent experiments. In panel B, *p < 0.05 vs. shScr, #P< 0.05 vs. shc-Myc. In panel G, *p< 0.05 vs. IgG control.

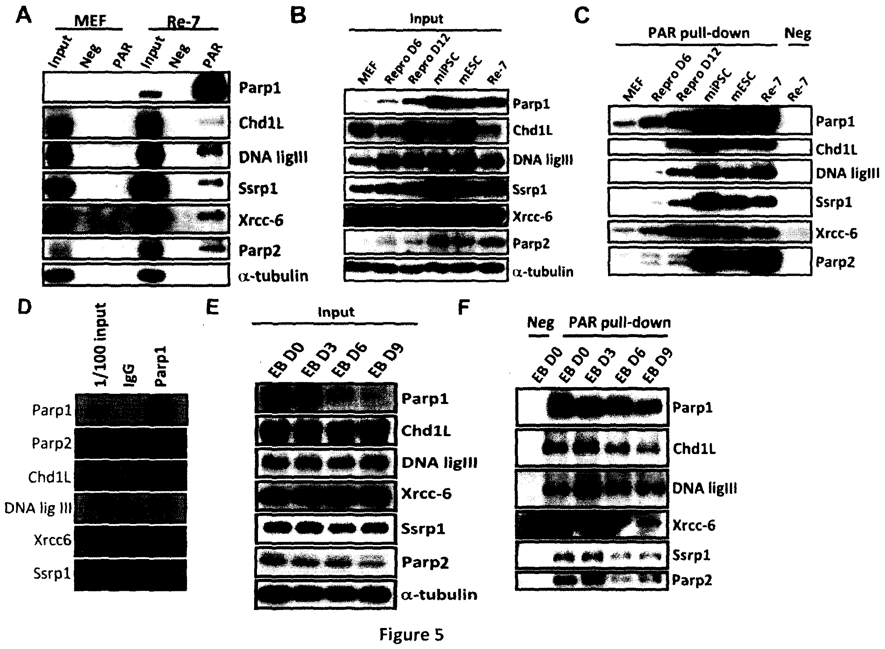

Figure 5 shows that Parp1 maintains the PARylation of chromatin-remodeling proteins in pluripotent stem cells. Panel A: PARylated proteins in iPSCs were prepared by PARylated protein pull-down assays and analyzed by LC-MS/MS. Western blotting confirms the expression of these identified PARylated proteins (Neg: negative resin with mutant affinity domain; PAR: PAR affinity resin).Panel B: In the total lysates (input), western blotting shows the expression levels of Parp1, Parp2, Chd1L, DNA ligase III, Ssrp1, and Xrcc6 in pluripotent stem cells and during the reprogramming process. Panel C: Using PAR affinity resin with pull-down assays, evaluation of the expression profiles of the identified PARylated proteins in MEFs, reprogramming D6 cells, reprogramming D12 cells, miPSCs, mESCs, and Re-7iPSCs (Con: negative resin with a mutant affinity domain).Panel D:Co-immunoprecipitation showed that that Parp1 interacts with Parp2, Chd1L, DNA ligase III, Xrcc-6, and Ssrp1.Panel E: The total protein from iPSC-derived EBs were evaluated. Panel F: The protein expression of Parp1, Chd1L, DNA ligase III, Ssrp1, Xrcc-6, and Parp2 during the differentiation process of iPSC-derived EBs.The western blots are representative of three separate experiments with independent cell preparations.

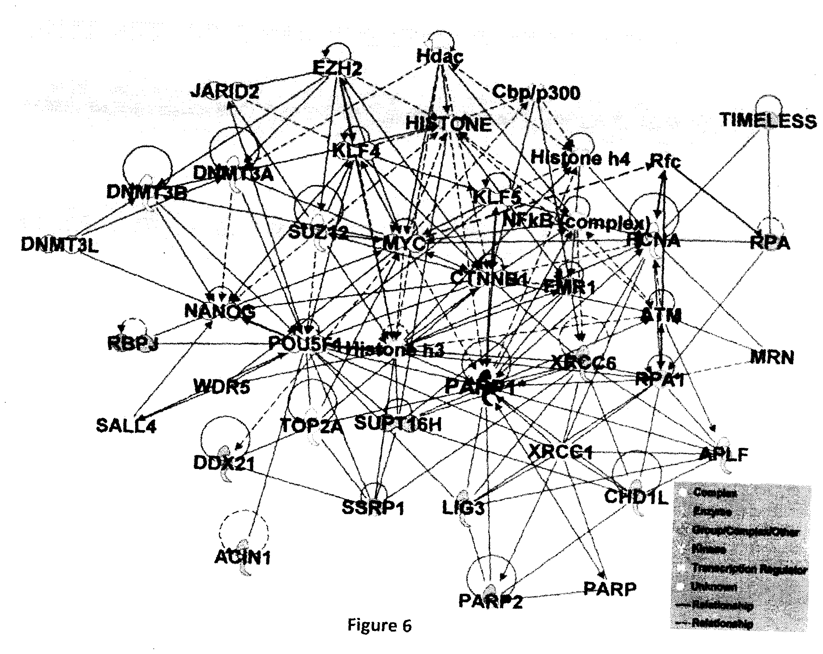

Figure 6 provides a diagram showing the protein-interaction network in nuclear reprogramming and pluripotency maintenance by Parp1-interacting and Parp1-PARylated proteinsusing Ingenuity IPA analysis.

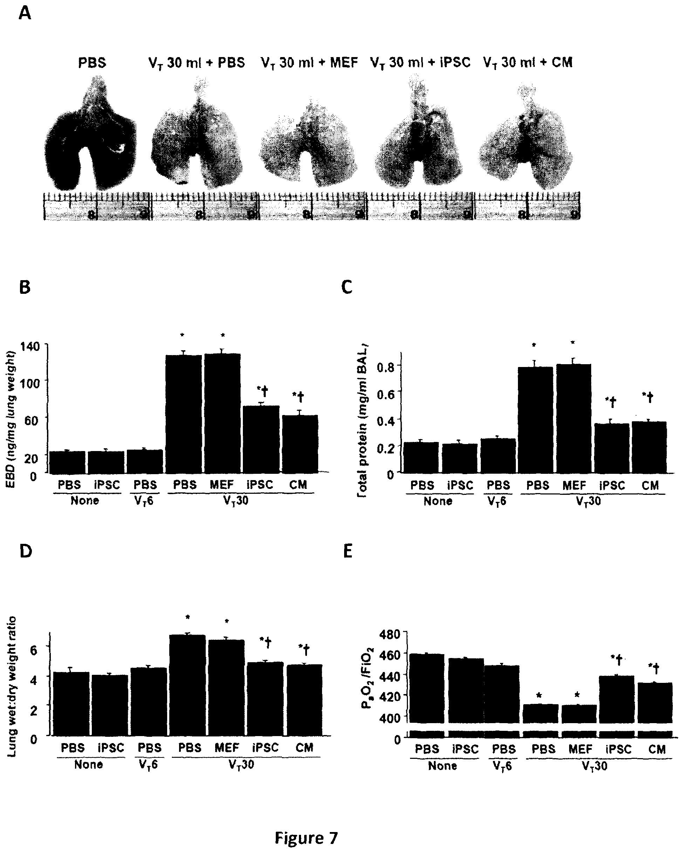

Figure 7 shows thatiPSCs or iPSC-CM ameliorated high tidal volume-induced lung edema and injury (VILI). Panel A provides gross pathologic images of lungs indicating high tidal volume- (V_{T}30) induced hemorrhaging, lungcongestion edema and the restorative effect of iPSCs or iPSC-CM. Panels B-E provide the effects ofadministering MEF, iPSC, or iPSC-CM onlung EBD (panel B), BAL total protein (panel C), wet-to-dry ratio(panel D), and PaO₂/FiO₂ ratio (panel E)in mice receiving mechanical ventilation at a low tidal volume (V_{T}6) or a high tidal volume (V_{T}30). Data shownhere are the mean ± SD of three independent experiments (*P< 0.05vs. non-ventilated control treated with PBS; †P< 0.05 vs. V_{T}30-ventilated mice treated withPBS).

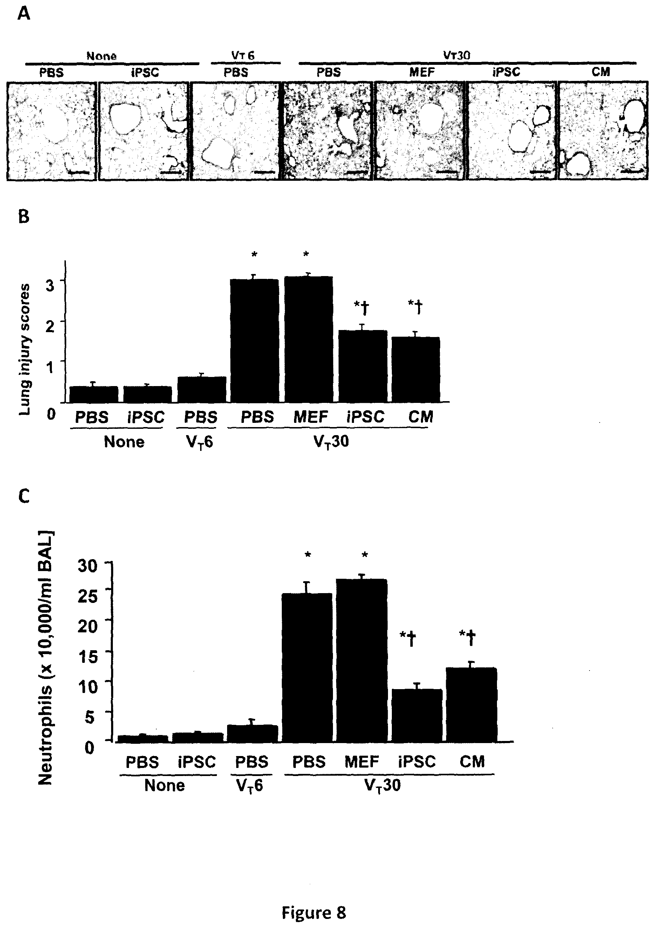

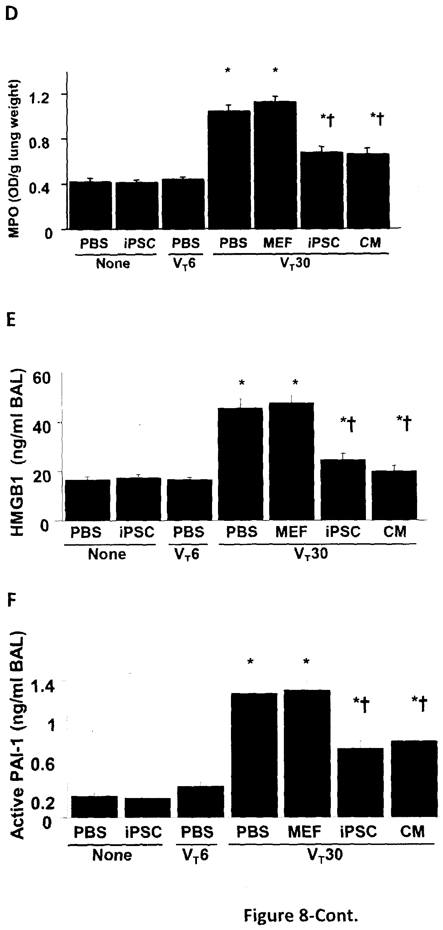

Figure 8 shows thatiPSCs or iPSC-CM restored high tidal volume-induced structural damage and neutrophil infiltration. PanelA provides histological examination;panelB provides the quantification of V_{T}30 induced airway structural damage and the restorative effect of iPSCs oriPSC-CM; and Panels C-F provide the effects of administering MEF, iPSC, or iPSC-CM on neutrophilinfiltration(panel C), MPO activity(panel D), HMGB1 secretion(panel E), and PAI-1 secretion (panel F)in bronchialalveolar lavage in mice receiving mechanical ventilation at a low tidal volume (V_{T}6) or a high tidal volume (V_{T}30). Data shown here are the mean ± SD of threeindependent experiments (*P< 0.05 vs. non-ventilated mice treated with PBS; †P< 0.05 vs. V_{T}30-ventilated mice treated with PBS).

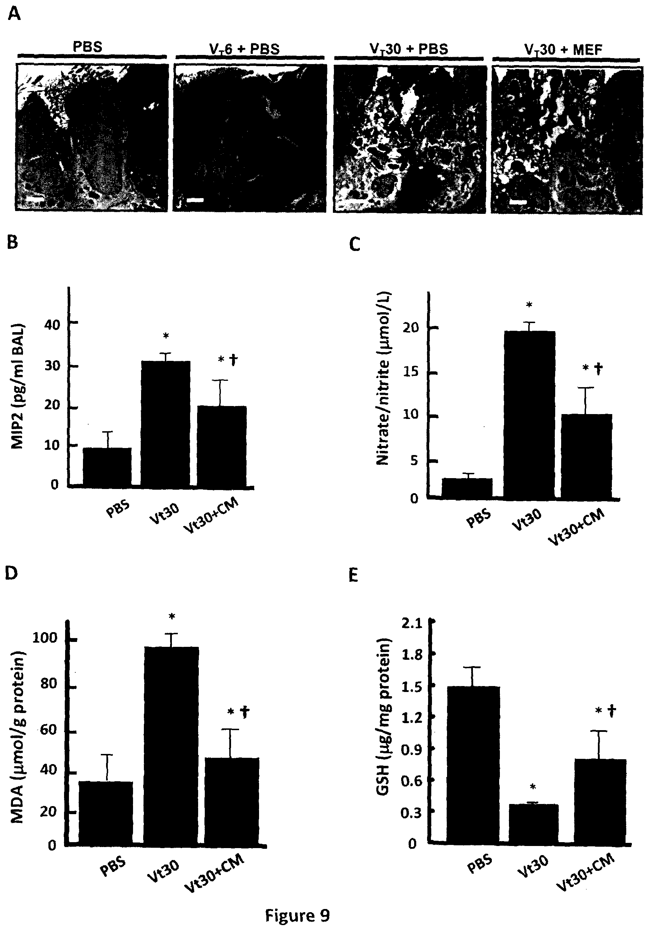

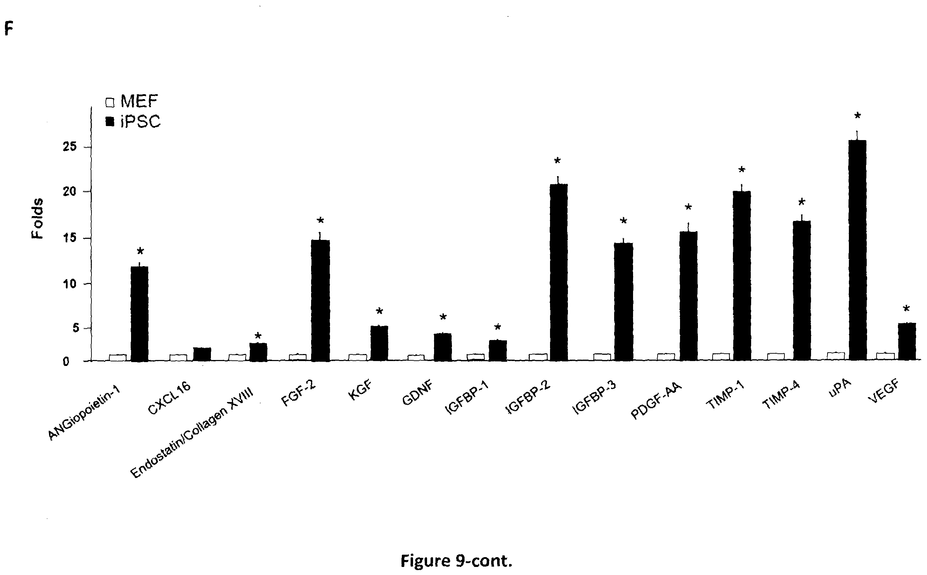

Figure 9 shows theultramicrostructural restoration by iPSC-CM. PanelA provides TEM images revealing there storation of airway ultramicrostructure by iPSCs or iPSC-CM treatment; Panels B-E provide the effects ofiPSC-CM administration on the expression of MIP2(panel B), the production of nitrate/nitrite(panel C),MDA(panel D), and GSH (panel E)in mice receiving V_{T}30 ventilation (data shown here are the mean ± SD of threeindependent experiments; *P< 0.05 vs. non-ventilated mice treated with PBS only (PBS); †P< 0.05 vs. V_{T}30-ventilated mice (Vt30)); and panel F provides the cytokine profile of iPSC-CM detected by a cytokine array.

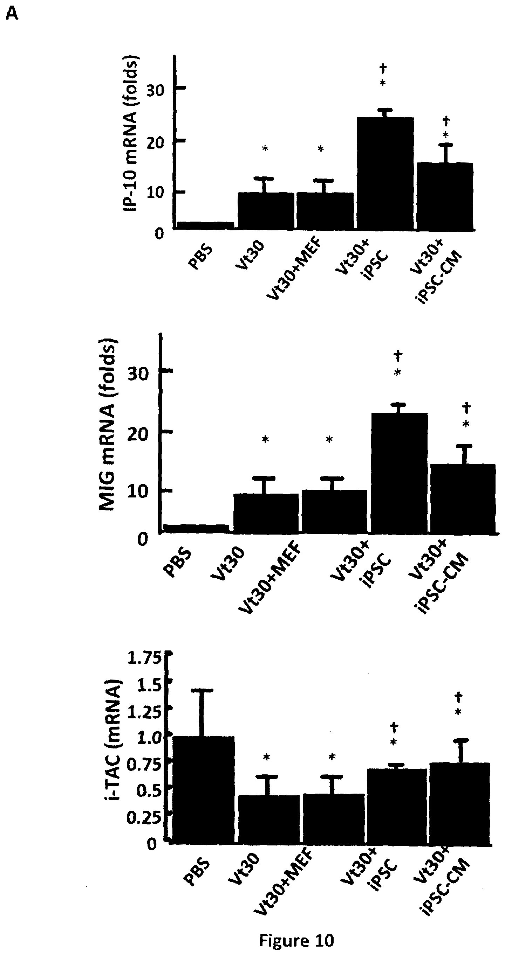

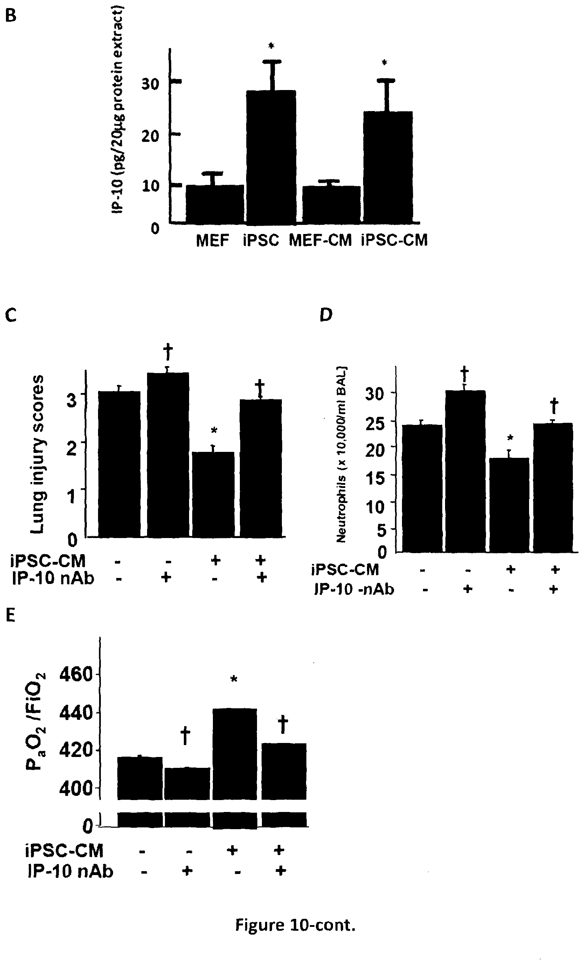

Figure 10 shows the involvement of IP-10 in the reparative effect of iPSC-CM in VILI. Panel A provides quantitative RT-PCR indicating that both iPSCs and iPSC-CM recruited the expression of IP-10 (top), MIG (middle), and i-TAC (bottom) in V_{T}30 ventilatedmice; panel B providesthe ELIZA data of IP-10 secretion stimulated by iPSCs in mice receiving V_{T}30 ventilation; and panels C-E provide the effects of IP-10 neutralizing antibody administration on lung injury scores(panel C), neutrophil infiltration(panel D), and PaO₂/FiO₂ ratio (panel E)in mice receiving V_{T}30 ventilation. Data shown here are the mean ± SD of three independent experiments. In panel A, *P< 0.05 vs.PBS only; †P< 0.05 vs. V_{T}30 only. In panel B, *P< 0.05 vs.MEF-treated mice. Inpanels C-E, *P< 0.05 vs. V_{T}30-ventilated mice without treatment; †P<0.05 vs. IP-10 neutralizing antibody-untreated group with or without iPSC-CM treatment.

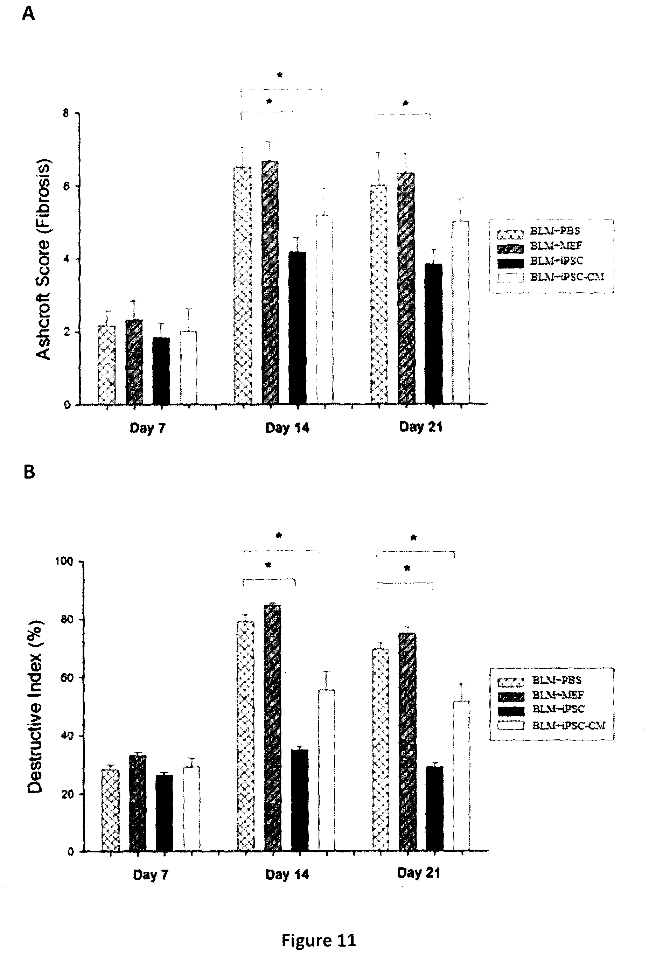

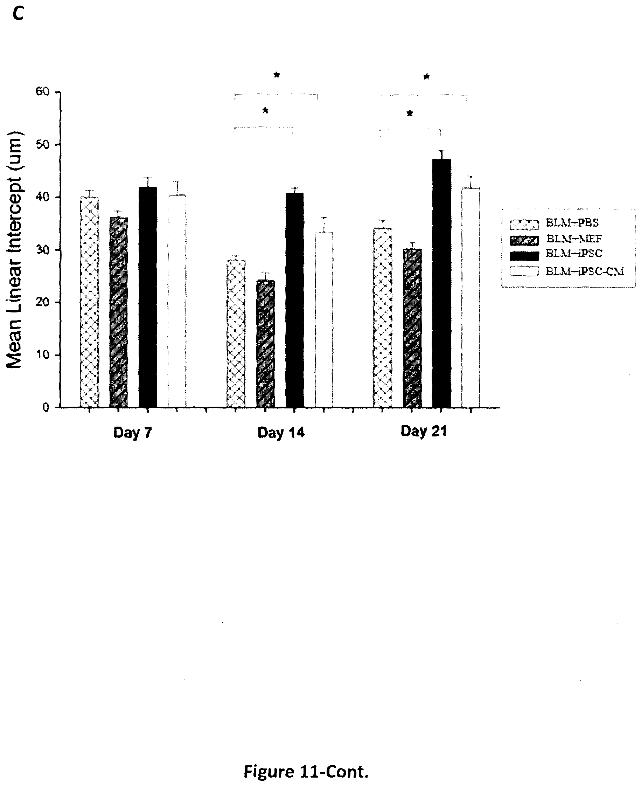

Figure 11 shows that intravenous transplantation of iPSCs or iPSC-CM improved lung injury followingbleomycin-induced pulmonary fibrosis. Panel A providesthe Ashcroft lung fibrosis score; and panels B-C provide the destructive index and the mean linear intercept (Lm) values, respectively, obtained at 7, 14, and 21 days following treatment with iPSCsor iPSC-CM. Data are expressed as the mean ± SD (*p<0.05 vs. bleomycin-treated PBS control atindicated day; n=6 per group).

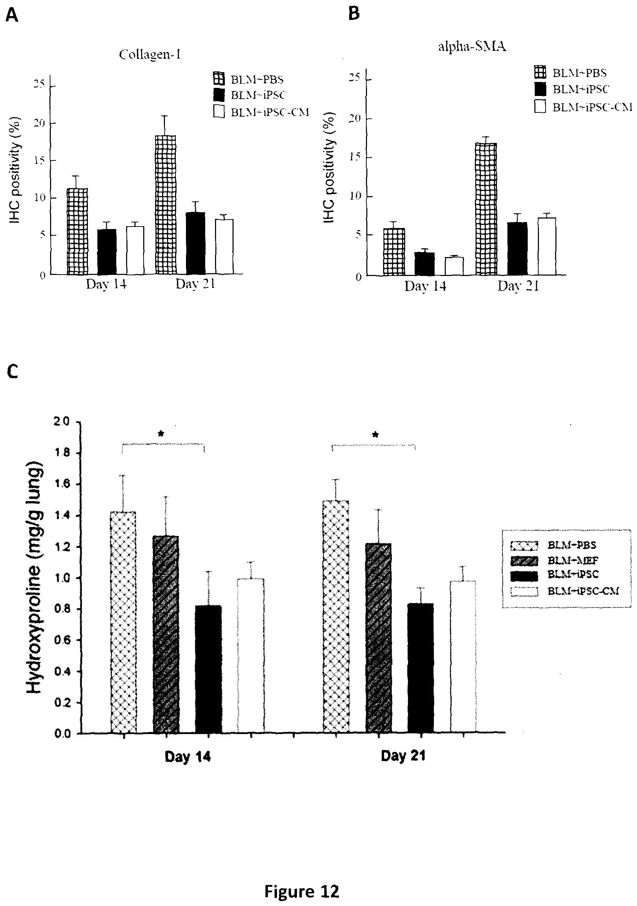

Figure 12 shows that intravenous transplantation of iPSCs or iPSC-CM attenuated the intensity ofbleomycin-induced pulmonary fibrosis.Panels A-B providequantification of type 1 collagen and α-SMA staining in bleomycin-treated mice receiving iPSCs or iPSC-CM; and panel Cprovides hydroxyproline content in lungs from mice that received iPSCs oriPSC-CM at 14 and 21 days after intra-tracheal bleomycin injection. Data are expressed as the mean±SD (*p<0.05 vs. bleomycin-treated PBS control at indicated day; n=5 per group).

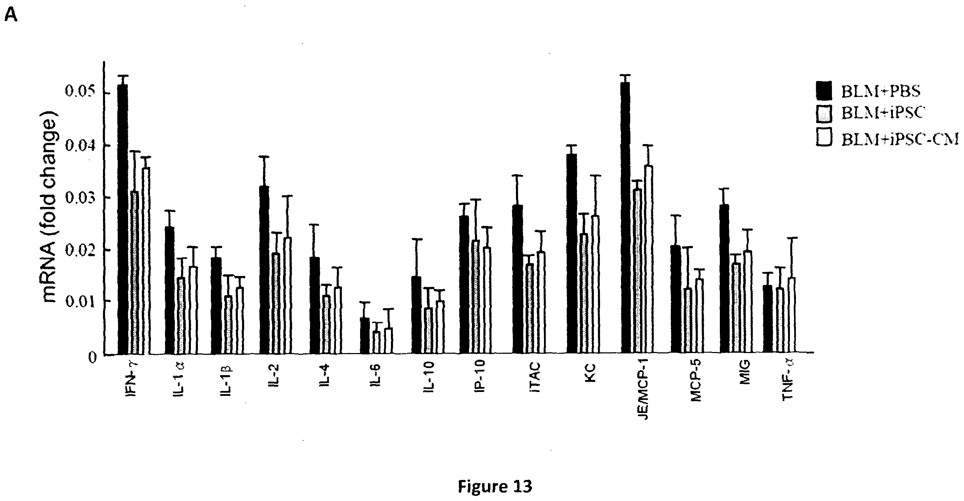

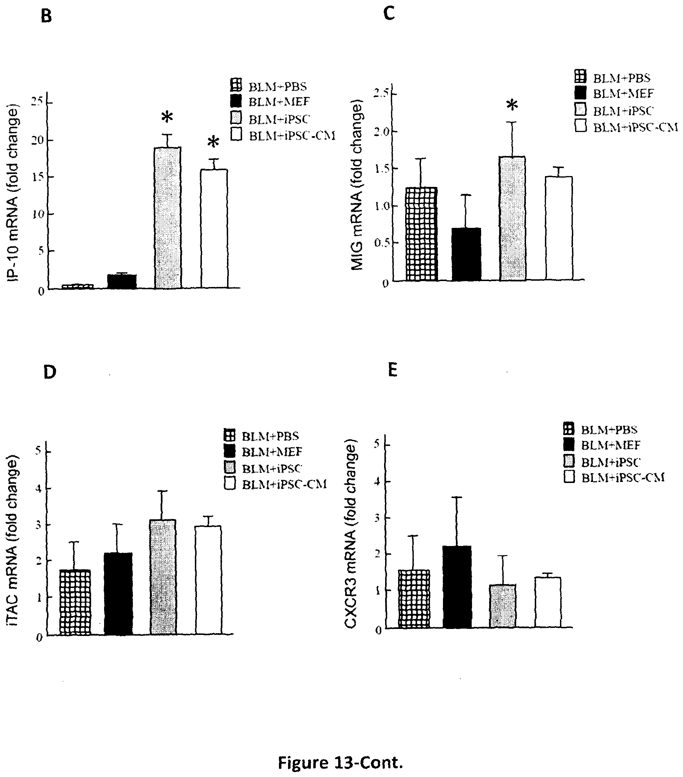

Figure 13 provideschanges in pulmonary cytokines and chemokines 3 days after iPSC treatment inbleomycin-injured mice.Panel A providesthe expression of individualcytokines and chemokines quantified by densitometry and expressed as pixel density, in which lung proteins from bleomycin-injured recipients of iPSCs oriPSC-CM were extracted and analyzed using a cytokine array assay kit; and panels B-E provide real-timePCR results for IP-10 (panel B), MIG (panel C), iTAC (panel D), and CXCR3 (panel E) extracted from whole lungs and analyzed 3 days after bleomycin-induced lung injury, in whichresults are expressed as the fold expression over PBS-treated lungs. Data are expressed as the mean±SD (*p<0.05 vs. bleomycin-treated PBS control; n=5per group).

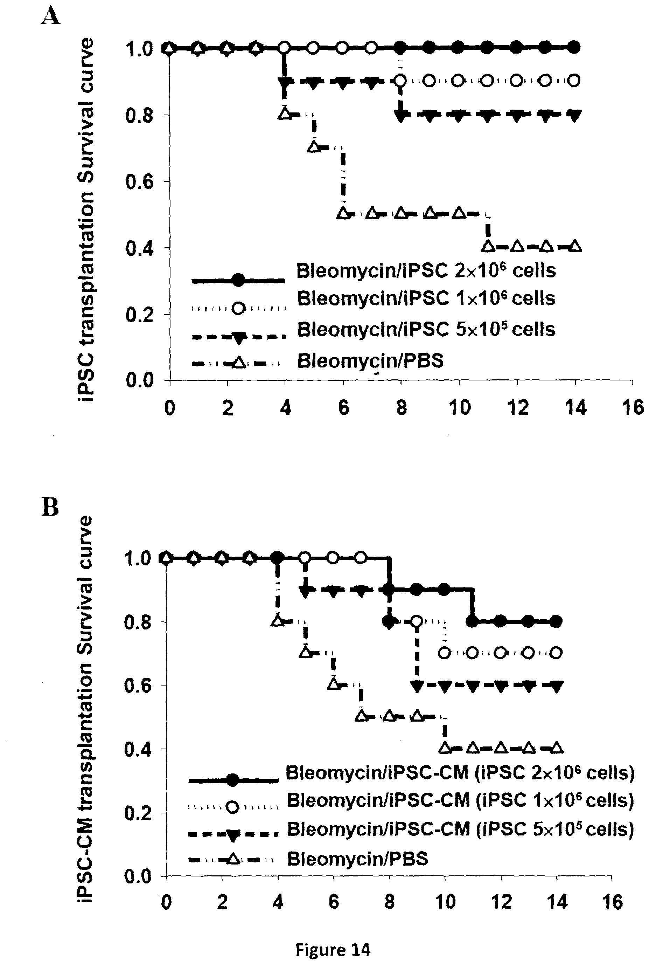

Figure 14 shows dose-related effects of iPSCs (panel A) or corresponding iPSC-CM (panel B) treatment on the survival of bleomycin-injected mic. Data are expressed as the mean ±SD (*p<0.05; n=5 per group).

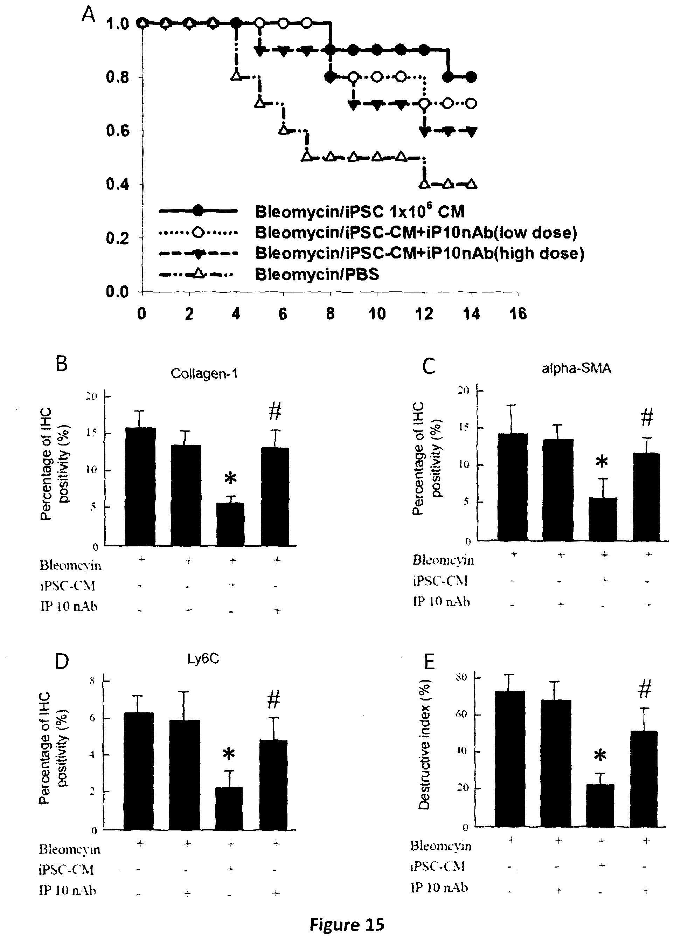

Figure 15 shows the involvement of IP-10 in the protective effect of iPSC-CM on bleomycin-inducedpulmonary fibrosis. Panel A provideseffects of IP-10-neutralizing antibody on iPSC-CM-mediated protection againstbleomycin-induced lethality; and panels B-E provide effect of IP-10-neutralizing antibody on the collagen deposition(panel B), myofibroblast accumulation(panel C), neutrophilaccumulation(panel D) and Ashcroft fibrosis score (panel E)measured in bleomycin-injured mice treated withiPSC-CM. Data are expressed as the mean±SD (*p<0.05 vs. bleomycin-treated PBS; #p<0.05 vs.bleomycin-treated iPSC-CM group; n=5 per group).

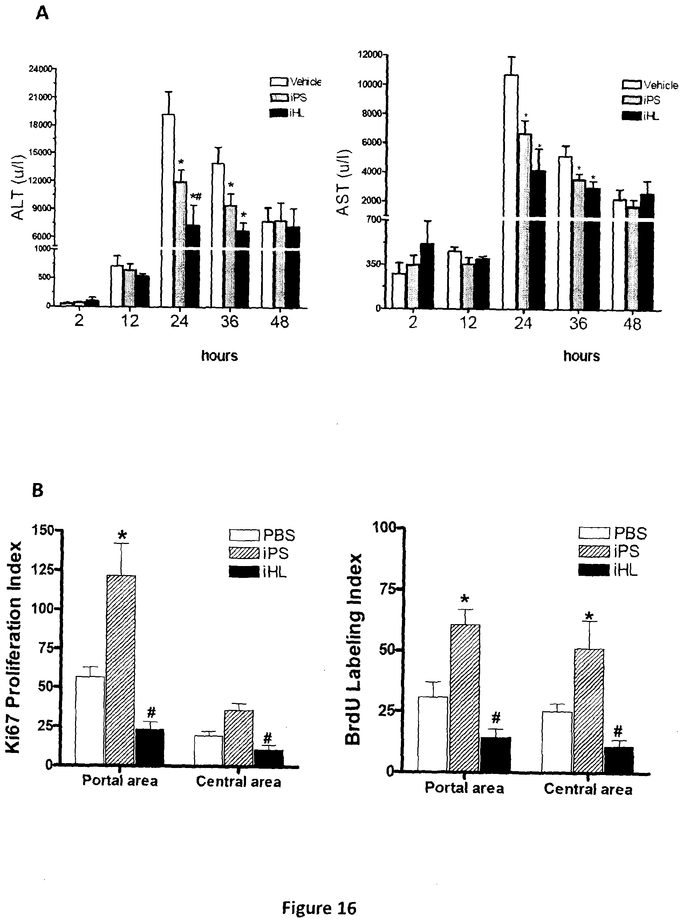

Figure 16 showsthat iPS cells alleviated liver injury and reduced liver tissue damage.Panel A providesthe serum levels of liver enzymes (ALT, AST) in mice receiving either vehicle (PBS),iPS, or iHL cell infusion, which was determined at different time points after injection of CCl4 (n=6, *P < 0.05 vs. vehicle, # P < 0.05 vs. iPS); and panel B provides Ki67 proliferation index and BrdU labelling index of the indicated groupsmeasured at 48 h post CCl4 treatment (n=6, *p<0.05 vs. PBS, #p<0.05 vs. iPS).iPS, induced pluripotentstem cells; iHL, iPS-derived hepatocyte-like cells; ALT, alanine aminotransferase; AST, aspartateaminotransferase; CCl4, carbon tetrachloride.

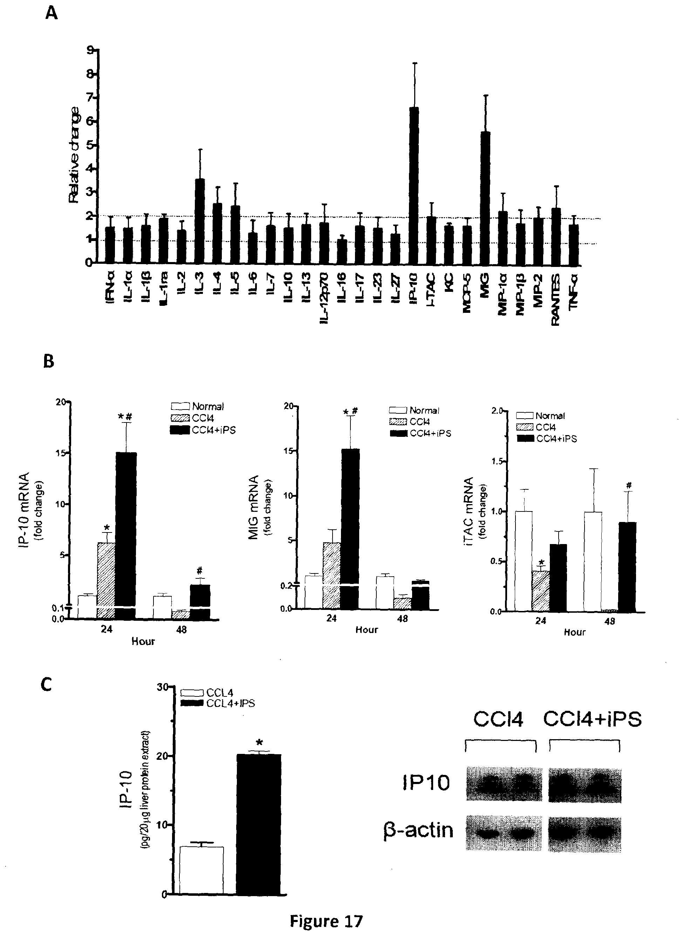

Figure 17 provides the changes of hepatic chemokines after iPS infusion in injured mice. Panel A provides the iPS-inducedhepatic cytokine profile evaluated by cytokine arrays, in which liver proteins were extracted and analysed by cytokine array assay kit, and the expression of individual cytokine was quantified by densitometry and expressed as fold change relative to the group of CCl4-injury without iPS cells; panel B shows the effects of iPS cells on the mRNA expression of IP-10, MIG and iTACafter CCl4 injury (n=6, *p<0.05 vs. normal control,#p<0.05 vs. CCl4 group); and panel C provides the ELISA and western blot analyses of hepatic IP-10 in homogenized liverextracts at 24 h post-injury (n=4, *p<0.05 vs. CCl4 group).

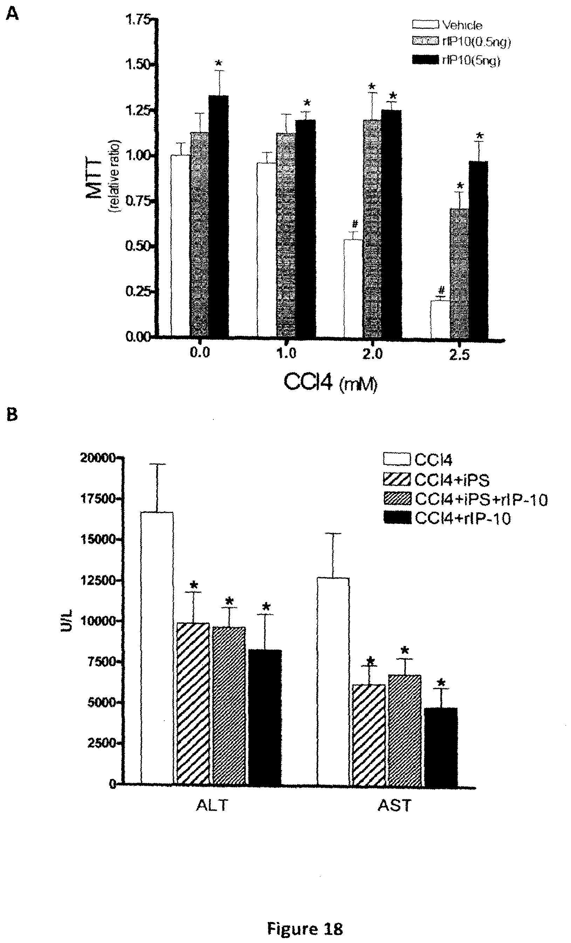

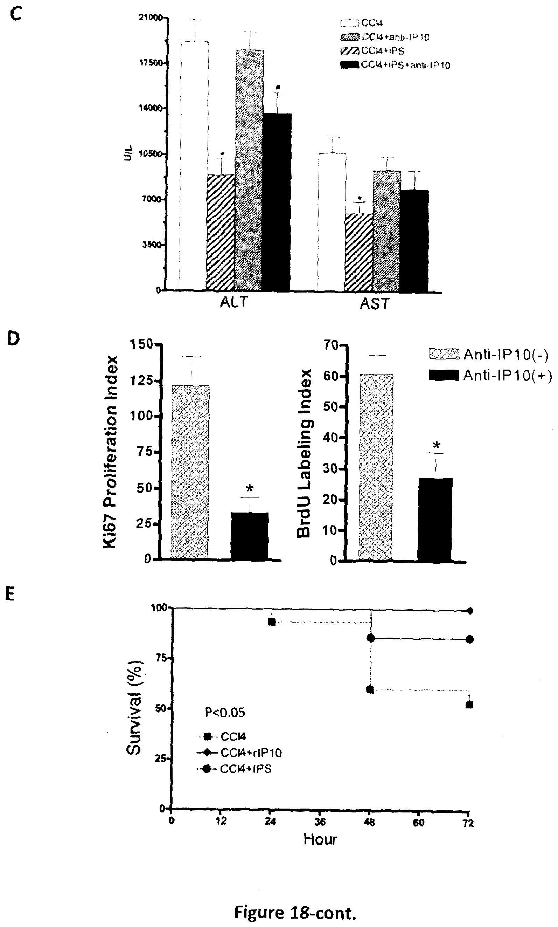

Figure 18 shows that IP10 is an important factor mediating the beneficial effects of iPS.Panel A provides the effects of recombinant IP 10 (rIP-10) increasing the viability of injured hepatocytes 24 h after CC14 injury at concentrationof 1.0 to 2.5 mM; panel B shows the protective effects of rIP-10 on liver damage; panel C demonstrates the effects of anti-IP-10 neutralizing antibody inattenuating the protective effects of iPS cells (n=6, *p<0.05 vs. CCl4 group,#p<0.05 vs. CCl4+iPS group); panel D demonstrates that anti-IP-10 neutralizing antibody reduced the proliferation of hepatocytes at portal regions after iPS infusion; and panel E provides the survival curve of mice treated with CCl4, CC14+iPS cells or rIP10(*p<0.05, n=6 in each group).

### DETAILED DESCRIPTION OF THE INVENTION

Unless defined otherwise, all technical and scientific terms used herein have the same meaning as commonly understood by a person skilled in the art to which this invention belongs.

As used herein, the article "a" or "an" means one or more than one (that is, at least one) of the grammatical object of the article, unless otherwise made clear in the specific use of the article in only a singular sense.

The term "pluripotent" as used herein refers to a cell with the capacity, under different conditions, to differentiate to more than one differentiated cell type, and preferably to differentiate to cell types characteristic of all three germ cell layers.

As used herein, the terms "iPSC" and "induced pluripotent stem cell" are used interchangeably and refers to a pluripotent stem cell artificially derived (e.g., induced or by complete reversal) from a non-pluripotent cell, typically an adult somatic cell, for example, by inducing a forced expression of one or more genes.

This invention is based on the finding that Parp1 can be used in place of c-Myc and Klf4 in the reprogramming process and the efficiency thereof was remarkablysimilar to those reprogrammed by c-Myc,Oct4 and Sox2 (OSM)in terms of production of iPSC. Moreover, several Parp1-associated and PARylation-interacted proteins in iPSCs and ESCs were also identified in the invention, including those involved in DNA repair and chromatin re-openning, thereby providing a useful tool for iPSC technology and regenerative medicine for aged cells or tissues.

Poly(ADP-ribose) polymerase 1 (Parp1), a member of the Parp family of proteins, is a highly conserved DNA-binding protein that is abundant in the nucleus. Parp1 is known as a key effector of several nuclear events such as DNA repair, replication, and transcription (Jagtap and Szabo, 2005; Kraus, 2008). It catalyzes a process called poly(ADP-ribosylation) (PARylation), in which NAD⁺ is used as substrate to synthesize poly(ADP-ribose) polymers with sizes varying from 2 to 200 ADP-ribose units (Krishnakumar and Kraus, 2010). This Parp1-catalyzed PARylation has been implicated in several processes, including chromatin remodeling, enhancer binding, coregulation, and insulation (Kraus, 2008).

In one aspect, the present invention provides a method as defined in the claims for preparing induced pluripotent stem cells (iPSCs) from somatic cells, comprising: (a) transfecting isolated somatic cells to express Oct3/4, Sox2, and Parp1; and (b) culturing the transfected somatic cells as obtained in step (a) under appropriate conditions, thereby converting the somatic cells into iPSCs and maintaining pluripotency and self-renewal ability.

The isolated somatic cells are transfected with one or more plasmid or viral vectors comprising Oct3/4, Sox2, and Parploperably linked to a promoter.

In another aspect, the present invention provides a method for preparing induced pluripotent stem cell (iPSCs) from somatic cells, comprising: (a) contacting or exposing isolated somatic cells with Oct3/4, Sox2, and Parp1, and (b) culturing the somatic cells as obtained in step (a) under appropriate conditions, thereby converting, at least a subset of, the population of somatic cells into iPSCs and maintaining pluripotency and self-renewal ability. The invention is characterized in that the method does not comprise a step of transfecting, contacting, or exposing the somatic cells to with/to c-Myc, K1f4, Nanog, Lin28, or any combination thereof, distinguish from the prior art.

Also disclosed in this description is an iPSC produced by the method described herein.

Also provided in the invention is a method as defined in the claims of preparing iPSCs, which comprises (a) providing isolated somatic cells; (b) transfecting, contacting or exposing the isolated somatic cells with/to express Oct3/4, Sox2, and Parp1; and (c) culturing the somatic cells as obtained in step (b) under appropriate conditions, thereby converting the somatic cells into iPSCs and maintaining pluripotency and self-renewal ability.

The present description also discloses a method of reprogramming adult cells comprising administering the cells with a protein that is/can be PARylated. Furthermore, it is believed that the cell rejuvenation can be performed by the reprogramming process. Accordingly, the present description discloses a method of rejuvenating cells or senescent cells in a subject, comprising administering the cells or the subject with a protein that is/can be PARylated.

The term "reprogramming" used herein refers to a process of erasure and remodeling of epigenetic marks, such as DNA methylation wherein the original DNA methylation patterns are erased and re-established.

The phrase"rejuvenation of cells"used herein refers to a process for rendering the telomere size, gene expression profiles, oxidative stress, or mitochondrial metabolism of such cells indistinguishable from that of embryonic stem cells.

As used herein, the term "subject" refers to a human or a mammal, such as a patient, a companion animal (e.g., dog, cat, and the like), a farm animal (e.g., cow, sheep, pig, horse, and the like) or a laboratory animal (e.g., rat, mouse, guinea pig, and the like).

The protein that can be PARylated or is related to PARylation can be one selected from the group consisting of the proteins listed in the table below and a combination thereof:

| no | protein name |
|---|---|
| 1 | Poly [ADP-ribose] polymerase 1 |
| 2 | FACT complex subunit SPT16 |
| 3 | Poly [ADP-ribose] polymerase 2 |
| 4 | Chromodomain-helicase-DNA-binding protein 1-like |
| 5 | DNA ligase 3 |
| 6 | FACT complex subunit SSRP1 |
| 7 | Leucine-rich repeat flightless-interacting protein 2 |
| 8 | X-ray repair cross-complementing protein 6 |
| 9 | X-ray repair cross-complementing protein 1 |
| 10 | Splicing factor U2AF 35 kDa subunit |
| 11 | Protein timeless homolog |
| 12 | Nucleolar RNA helicase 2 |
| 13 | U1 small nuclear ribonucleoprotein A |
| 14 | Tyrosyl-DNA phosphodiesterase 1 |
| 15 | Aprataxin and PNK-like factor |
| 16 | Replication protein A 70 kDa DNA-binding subunit |
| 17 | Apoptotic chromatin condensation inducer in the nucleus |
| 18 | Heterogeneous nuclear ribonucleoprotein A3 |
| 19 | Fragile X mental retardation protein 1 homolog |
| 20 | Recombining binding protein suppressor of hairless |
| 21 | Splicing factor U2AF 65 kDa subunit |

The protein can be Chromodomain-helicase-DNA-binding protein 1-like (Chd1L).

An enzyme that has PARylation activity can also be used to reprogramming or rejuvenating adult cells. The enzyme can be Parp 1.

As used herein, the term "adult cell" refers to a cell found throughout the body after embryonic development.

In the present disclosure, the iPSCs or iPSC-CM thereof was demonstrated to have the efficacy to induce the production of IP-10 in injured tissues, such as injured lungs and livers (Fig. 10, 13 (panel A) and 17). Accordingly, the description discloses a method for inducing the secretion of IP-10 which comprisesadministering to a subject in need thereof an effective amount ofiPSCs oriPSC-CM.

As used herein, the term "iPSCs" or "iPS cells" refers to induced pluripotent stem cells which can be generated from an individual's somatic cells and are capable of self-renewal and differentiation into several different cell types after proper induction. The somatic cells as used herein may be from a human or a mammal.

As shown in the description, administration of IP-10-neutralizing antibodies increased neutrophil infiltration, impaired lung oxygenation and deteriorated the protective effects mediated by iPSC-CMin a ventilator-induced lung injury (VILI) model (Fig. 10). In another example, IP-10 neutralization blocked the positive effects of iPSC-CM treatment or iPSCtransplantation on the survival rate of bleomycin-injected mice (Fig. 15). In a further example, the application of IP-10 neutralizing antibody attenuated the protective effects of iPSCs on a CCl4-injured liver (Fig. 18).

Accordingly, the secretion of IP-10 in a subject induced by the method provided herein is in a level effective to repair tissue injuries, including but not limited to lung injuries, liver injuries, and those injuries caused by corneal damages, limb disorders, kidney diseases, and ischemia.
Examples of ischemia are focal stoke and myocardial infarction.

Given the protective effects of the increased level of IP-10 induced by iPSCs and iPSC-CM in injured tissues, the description discloses a method for treating tissue injuries, for example lung or liver injuries, which comprises administering to a subject in need thereof a therapeutically effective amount of iPSCs or iPSC-CM, wherein the therapeutically effective amount is an amount capable of inducing a sufficient level of IP-10 to suppress inflammation responses.

On the other hand, a composition comprising iPSCs or iPSC-CM for inducing the secretion of IP-10 in a subject is also disclosed in the present description.

The specific example below is to be construed as merely illustrative, and not limitative of the remainder of the disclosure in any way whatsoever. Information provided in the examples relating to subject-matter not encompassed by the claims is for reference. Without further elaboration, it is believed that one skilled in the art can, based on the description herein, utilize the present invention to its fullest extent.

### Examples- (I)

### I. Materials and Methods

### 1. Generation of iPSC lines and cell culture

C57/B6 mice were used in this study and all procedures involving animals were approved by the Animals Committee of the Taipei Veterans General Hospital. Murine iPSCs were generated from mouse embryonic fibroblasts (MEFs) derived from 13.5-day-old embryos of C57/B6 mice. The iPSCs were reprogrammed by the transduction of retroviral vectors encoding four transcription factors (Oct4/Sox2/Klf4/c-Myc; OSKM), or three transcription factors (Oct4/Sox2/Klf4; OSK), as described previously (Li et al., 2011). Total of 12 clones (Re-1 to Re-12; OSKM) were selected and established. The clone of Re-7 iPSCs had been stably passed to more than 100 th passages with high pluripotency. Therefore, Re-7 iPSCs were selected and widely used in this study. Briefly, undifferentiated iPSCs were routinely cultured and expanded on mitotically-inactivated MEFs (50,000 cells/cm 2) in six-well culture plates (BD Technology) in the presence of 0.3% leukemia inhibitory factor in an iPSC medium consisting of Dulbecco's Modified Eagle's Medium (DMEM; Sigma) supplemented with 15% fetal bovine serum (FBS; Invitrogen), 100 mM minimal essential medium (MEM) nonessential amino acids (Sigma), 0.55 mM 2-mercaptoethanol (Gibco), and antibiotics (Invitrogen). Every three to four days, colonies were detached with 0.2% collagenase IV (Invitrogen), dissociated into single cells with 0.025% trypsin (Sigma) and 0.1% chicken serum (Invitrogen) in PBS, and replated onto MEFs. For embryoid body (EB) formation, iPSCs were dissociated into a single cell suspension by 0.25% trypsin-EDTA and plated onto non-adherent culture dishes in DMEM with 15% FBS, 100 mM MEM nonessential amino acids, 0.55 mM 2-mercaptoethanol, and antibiotics at a density of 2x10 6 cells/100 mm plate. After 4 days in floating culture, EBs were transferred onto gelatin-coated plates and maintained in the same medium for 24 h. EBs werethen assigned for in vitro differentiation into tridermal lineages as previously described (Li et al., 2011).

### 2.Alkaline phosphatase activity (ALP), Alizarin red and PAS staining

For detecting the AP activity of cells on original plates, cells were fixed with 80% alcohol, and then fixed cells were stained using the Vector Blue Alkaline Phosphatase Substrate Kit III (Vector Laboratories) according to the manufacturer's instructions. Alizarin red staining and PAS staining was performed as previously described (Li et al., 2011).

### 3. One-dimensional gel electrophoresis (SDS-PAGE) and in-gel digestion

Of every cell type, extracted nuclear proteins ofl 0 ⁷cells from each condition were denatured by boiling at 95 °C for 10 min. 1D gel electrophoresis was described as previously (Liu et al., 2012), and performed with 10% SDS-PAGE. After the gel was stained by VisPRO 5 minutes protein stain kit (VP01-500, Visual Protein, Taipei, R.O.C.), every lane was cut off into 10 equal sections followed by reduction with β-Mercaptoethanol (1% v/v) in 25 mM ammonium bicarbonate at room temperature in the dark for 20 min, and alkylation with 5% v/v 4-Vinylpyridine in 25 mM ammonium bicarbonate for 20 min. Digestion was then done with 0.1% v/v proteomics grade modified trypsin (Sigma, Missouri, USA) in 25 mM ammonium bicarbonate at 37 °C overnight. Extracts of trypsin digested peptides were dried in a SpeedVac concentrator (Jouan, RC1022, Thermo Savant, Milford, MA, USA).

### 4. LC-MS/MS analysis

LC-MS/MS analysis was performed through the application of LTQ Orbitrap (Thermo Fisher Scientific Inc., Bremen, Germany) as described. In breif, each sample of digested peptides was reconstituted to 20 µl of 0.1% formic acid (FA). Peptides were firstly injected in and separated by the nanoflow HPLC (Agilent 1100, Agilent Technologies, Santa Clara, CA, USA) with a C18 column (75 µm ID × 360 µm OD × 15 cm; Agilent Technologies, Santa Clara, CA, USA), and became ionized particles once passed through the succeeding nanospray tip (New Objective, Woburn, MA). In operating HPLC, the flow rate was at 0.4 µl/min after a splitter. LC gradient for the LC-MS/MS system ramped from 2% ACN to 40% ACN in 120 min, and the system was performed under the setting of automated data-dependent acquisition, with mode of 200-2000 m/z full scan for the maximum 3 most intense peaks from each Orbitrap MS scan. Peptides with +2 or +3 charge state were further was subjected to CID. Spectra were obtained in raw data files with Xcalibur (version 2.0 SR2). Protein identification was accomplished via TurboSEQUEST (Thermo Finnigan, San Jose, CA, USA) using the UniProt database. A protein was confirmed once 3 peptides with Xcorr > 2.5 were matched in sequencing.

### 5. Parylated protein purification

Since parylated proteins contain poly-ADP-ribose that has high affinity with macrodomains, the PAR affinity resin set (Tulip) conjugated Af1521 macrodomains was used to pulldown the parylated proteins. Protein was extracted from cells by lysis buffer (e.g.: 50mM Tris, pH 8, 200mM NaCl, 1mM EDTA, 1% Triton X-100, 10% glycerol, 1 mM DTT, 0.5% deoxycholate, and protease inhibitors) and incubated with resin overnight in 4°C. After incubation, resin was washed with lysis buffer three times followed by addition of IX SDS-sample buffer at 95°C for 10 min to dissociated proteins.

### 6. Gene ontology, pathway, and network analysis

The filtered genes and proteins were subjected to gene ontology enrichment analysis using the AltAnalyze bundled module GO-Elite (http://www.genmapp.org/go_elite). GO-Elite implements an over-representation statistical inference that can identify significantly enriched GO categories with nuclear proteins. GO terms with a z-score >2, a permutation of P<0.01 and three or more regulated proteins for each GO term were reported as significant. Canonical pathway and gene interaction network analyses were conducted using Ingenuity Pathway Analysis web tool (IPA, http.//www.ingenuity.com/). IPA constructs hypothetical protein interaction clusters on the foundation of a regularly updated Ingenuity Pathways Knowledge Base, which is a database that consists of millions of individual relationships between proteins collected from the public literature. Every gene interaction is supported by evidence extracted from the underlying publications, structured using an ontology, and stored in the database. To further explore the potential relationship between the target-PARylated proteins (listed in Table 1) and stemness genes or other factors in regulating cellular reprogramming, the bioinformatics analysis of Ingenuity Pathway Analysis web tool (IPA, http://www.ingenuity.com/) was used in this study. Firstly, we uploaded a data set (contained the PARylated proteins identified in the proteomic experiment (Table 1)) into IPA web. Then we chose the option "New core analysis" to generate protein-protein interaction networks. We further used "Experimentally Observed" to limit the confidence, set reference as "Ingenuity Knowledge Base (Gene Only)" and output 10 networks (70 molecules per network). Hypothetical networks in Fig. 6 were generated from these target-PARylated proteins, ESC-related proteins, and other candidate proteins from the database by IPA analysis. The solid line is representative with the "Direct" relationship of protein-to-protein interaction supported by literature data base, while the dashed line is representative with "Indirect" interaction supported by literature data base.

### 7. Quantitative PCR and RT-PCR for marker genes

Reverse transcription reactions were performed using SuperScript TM IIIReverse Transcriptase (Invitrogen). cDNA was used in the following quantitative PCR (qPCR) and RT-PCR. qPCR was performed with Power SYBR Green PCR Master Mix (Applied Biosystems) according to manufacturer's instructions. Signals were detected with 7900HT Fast Real-Time PCR System (Applied Biosystems).

### 8. shLuc and shParp1 and shParp2 expression construct and lentiviral transduction

The stable ablation of Parp1 and Parp2 in MEFs was obtained using small hairpin RNA (shRNA) probes for the mouse gene Parp1 and Parp2. Control cells were allowed to stably express shLuc (pLKO.1-shLuc). Cells were infected with shRNA lentiviral-vector generated using a three-plasmid-based lentiviral system (all plasmids are available from the RNAi Consortium [TRC]). Lentivirus production was performed by transfection of 293T cells at 5x10⁶ cells per 10 cm plate using Lipofectamine 2000 (LF2000, Invitrogen Life Technologies, Carlsbad, CA, USA). Supernatants were collected 48 h after transfection and then were filtered. Subconfluent cells were infected with lentiviral-vector in the presence of 8 mg/ml polybrene (Sigma). Infected cells were selected with puromycin (2 mg/ml) until control uninfected cells were completely dead. Immunoblotting was used to confirm the knockdown efficiency of shParp1 and shParp2 (Chen et al., 2011).

### 9. Luciferase activity assay

STO cells were grown in 24-well tissue culture dishes to 70% confluence and then cotransfected with 0.2 µg of pMXs and pMXs-c-Myc in the presence of 0.2 µg of pGL3-PARP promoter firefly luciferase or PARP promoter mutants and 10 ng of SV40 Renilla luciferase plasmids (Promega). Twenty-four hours post-transfection, cells were harvested in 100 µlreporter lysis buffer and then subjected to a dual luciferase assay according to the manufacturer's protocol (Dual-Luciferase Reporter Assay System, Promega). Firefly luciferase activity was normalized to Renilla luciferase activity, and data are represented as the mean standard deviation of three independent experiments, each performed in triplicate.

### 10. Chromatin Immunoprecipitation (ChIP) and site-directed mutagenesis of mouse PARP promoter mutants

The study protocol of chromatin immunoprecipitation (ChIP) was according to the manufacturer's instructions (EZ ChIP kit, Upstate) using anti-c-Myc (sc-764, Santa Cruz) antibodies. The mouse PARP promoter with deletion or point mutation clones were created by site-directed mutagenesis according to the manufacturer's instructions (Phusion Site-Directed Mutagenesis Kit, Finnzymes), and all mutants were amplified by using PARP (-2000) as the template. Amplified fragments were further amplified with suitable forward primers with MluI cutting sequences and HIndIII cutting sequences in the reverse primers. Then, the restriction cutting site-added PCR products were subcloned into pGL3 luciferase reporter plasmids. DNA eluted from precipitated complexes in ChIP assays was amplified for the fragment of PARP promoter. As a positive control for ChIP study, c-Myc bound to its reported target, cyclin D2 promoter (Bouchard et al., 1999).

### 11. Western blot analysis and immunofluorescence staining

Western blot was performed as previously described (Li et al., 2011). For immunostaining, cells were cultured on cover slips, fixed with 4% paraformaldehyde, and permeabilized with 0.5% Triton X-100. Cells were stained with monoclonal anti-SSEAl antibody (Abcam) and anti-Oct4 monoclonal antibody (Cell Signaling), and then incubated with fluorophore labeledsecondary antibodies (Jackson Immunoresearch), Hoechst and DAPI (Sigma) before visualized under an Olympus microscope.

### 12. Chimera mouse production by blastocyst injection

The introduction of mouse iPS cells (derived from C57BL/6J strain, black coat color) into mouse blastocystsfrom C57BL/6J-Tyrc2J strain (albino) was performed as previously described with some modifications (Sung et al., 2006). The adult chimeras were confirmed by coat color, demonstrating that iPSCs were competent to produce adult chimeric mice. This study was assisted by Transgenic Mouse Model Core Facility, Academic Sinica, Taiwan.

### 13. Statistical analyses

Results are reported as mean ± SD. Statistical analysis was performed using Student's t test or a one-way or two-way analysis of variance (ANOVA) followed by Turkey's test, as appropriate. The survival rate analysis was performed using log-rank test. Results were considered statistically significant at P< 0.05.

### II. Results

### 1. Increased Parp1 and PARylation activity in reprogramming and pluripotent cells

Recent studies using MS-based proteomic analysis have confirmed the significant similarity between the proteomic profiles of iPSCs and ESCs (Jin et al., 2011; Munoz et al., 2011; Phanstiel et al., 2011). However, these studies were conducted with whole-cell lysates and did not focus on the differential regulation of nuclear events. In our previous work, we generated mouse iPSCs by overexpressing four genes Oct4/Sox2/Klf4/c-Myc (OSKM; Re-7 iPSC clone) or three genes (OSK without c-Myc) (Li et al., 2011). To distinguish the differences in the profiles of nuclear proteins between somatic and reprogrammed pluripotent cells, nuclear protein extracts from MEFs and Re-7 iPSCs were prepared. These extracts were then separated into five fractions by SDS-PAGE (Fig. 1, panel A). First, we established the differential expression profiles of these nuclear extracts using ID LC-MS/MS. Based on Gene Ontology (GO) database analysis, the predominant processes upregulated in the nuclear protein profiles of iPSCs included those pertaining to RNA processing, chromatin packaging and remodeling, cell structure and motility, and protein biosynthesis, as well as those involved in mRNA transcription and DNA replication (Fig. 1, panel B). Furthermore, using a statistical comparative analysis between the databases of ID LC-MS/MS and 2D-differential gel electrophoresis, we identified the 112 most upregulated nuclear proteins in iPSCs compared to MEFs.

Both ESCs and iPSCs maintain their genomic stability and pluripotency by enhancing DNA repair and NHEJ activity, and high levels of expression of DNA repair proteins, including Parp1, DNA ligIII, Rad51, and XLF, have been found in both ESCs and iPSCs (Fan et al., 2011). An elegant study provided by Doege et al. showed that Parp1 is involved in epigenetic modifications that direct subsequent transcriptional induction at pluripotency loci during somatic cell reprogramming (Doege et al., 2012). Using proteomic analysis and western blotting (Fig. 1, panel C), we found high Parp1 expression levels in the nuclear lysates of iPSCs, but not MEFs. One of the extensively characterized functions of Parp1 is the posttranslational modification of target proteins by attaching a poly(ADP-ribose) chain (PARylation) (Krishnakumar and Kraus, 2010). Using poly(ADP-ribose) affinity resin to pull down the PARylated proteins, we further demonstrated that Parp1 is the most highly expressed PARylaed protein in iPSCs compared to MEFs (Table 1).

**Table 1:**

| no | protein name | accession | gene name | MW (kDa) / pl | pep # (unique) | PEP |
|---|---|---|---|---|---|---|
| 1 | Poly [ADP-ribose] polymerase 1 | P11103 | Parp1 | 113.10 / 9.05 | 70(17) | <1.00E-307 |
| 2 | FACT complex subunit SPT16 | Q920B9 | Supt16h | 119.82 / 5.50 | 16(16) | 5.92E-148 |
| 3 | Poly [ADP-ribose] polymerase 2 | Q88554 | Parp2 | 63.40 / 8.65 | 15(15) | 3.28E-134 |
| 4 | Chromodomain-helicase-DNA-binding protein 1-like | Q9CXF7 | Chd1I | 101.44 / 6.20 | 11(11) | 1.54E-54 |
| 5 | DNA ligase 3 | P97386 | Lig3 | 113.07 / 9.16 | 10(10) | 3.32E-104 |
| 6 | FACT complex subunit SSRP1 | Q08943 | Ssrp1 | 80.86 / 6.33 | 4(4) | 9.96E-09 |
| 7 | Leucine-rich repeat flightless-interacting protein 2 | Q91WK0 | Lrrfip2 | 47.15 / 5.54 | 3(3) | 4.01E-61 |
| 8 | X-ray repair cross-complementing protein 6 | P23475 | Xrcc6 | 69.48 / 6.35 | 3(3) | 2.00E-10 |
| 9 | X-ray repair cross-complementing protein 1 | Q60596 | Xrcc1 | 68.97 / 5.97 | 3(3) | 8.30E-07 |
| 10 | Splicing factor U2AF 35 kDa subunit | Q9D883 | U2af1 | 27.82 / 9.09 | 2(2) | 3.35E-34 |
| 11 | Protein timeless homolog | Q9R1X4 | Timeless | 137.50 / 5.35 | 2(2) | 1.11E-22 |
| 12 | Nucleolar RNA helicase 2 | Q9JIKS | Ddx21 | 93.55 / 9.19 | 2(2) | 3.57E-05 |
| 13 | U1 small nuclear ribonucleoprotein A | Q62189 | Snrpa | 31.84 / 9.81 | 2(2) | 3.52E-04 |
| 14 | Tyrosyl-DNA phosphodiesterase 1 | Q8BJ37 | Tdpl | 68.69 / 7.67 | 2(2) | 1.81E-04 |
| 15 | Aprataxin and PNK-like factor | Q9D842 | Aplf | 54.97 / 5.05 | 2(2) | 2.55E-03 |
| 16 | Replication protein A 70 kDa DNA-binding subunit | Q8VEE4 | Rpa1 | 69.04 / 8.13 | 2(2) | 2.24E-03 |
| 17 | Apoptotic chromatin condensation inducer in the nucleus | Q9JIX8 | Acin1 | 150.72 / 5.71 | 1(1) | 3.75E-10 |
| 18 | Heterogeneous nuclear ribonucleoprotein A3 | Q8BG05 | Hnrnpa3 | 39.6S / 9.10 | 1(1) | 5.56E-05 |
| 19 | Fragile X mental retardation protein 1 homolog | P35922 | Fmr1 | 68.99 / 7.27 | 1(1) | 4.56E-03 |
| 20 | Recombining binding protein suppressor of hairless | P31266 | Rbpj | 58.54 / 8.43 | 1(1) | 2.56E-03 |
| 21 | Splicing factor U2AF 65 kDa subunit | P26369 | U2af2 | 53.52 / 9.19 | 1(1) | 1.14E-03 |

Therefore, we further attempted to elucidate whether Parp1 and PARylation may play a role in promoting cellular reprogramming and maintaining pluripotency. Notably, Parp1 protein, as well as Oct4, Nanog, and c-Myc, were upregulated in both whole-cell lysates and nuclear fractions of Re-7 iPSCs (Fig. 1, panel C). This upregulation of Parp1, accompanied by increased PARylation activity, was consistently observed in iPSCs generated with OSKM (Re-7 cells) or OSK, Dr. Yamanaka's iPSC clone (miPSCs) and ESCs (Fig. 1, panel C). Parp1 and PARylation, as well as these pluripotency factors, were completely undetectable in MEFs (Fig. 1, panel C). During the reprogramming process to convert MEFs to iPSCs, Parp1 and Oct4, Sox2, Nanog, and c-Myc were upregulated after the transfection of OSKM, and these proteins reached maximal expression 15 days after the induction of reprogramming (Fig. 1, panel D, upper). Increased PARylation activity was also observed during the reprogramming process (Fig. 1, panel D, lower). Furthermore, we analyzed whether PARylation was influenced by the differentiation of Re-7 iPSCs. Parallel to the downregulation of Parp1, the PARylation activity decreased significantly in iPSC-derived embryoid bodies (EBs) in a time-dependent manner (Fig. 1, panel E). Differentiation into different lineages was induced by specific protocols. Neuron-like, osteocyte-like (mesoderm), and hepatocyte-like (endoderm) cells were confirmed by immunofluorescence, Alizarin red and PAS staining, respectively (Fig. 1, panel F, left). After differentiation of Re-7 iPSCs into different lineages with each protocol, western blotting showed that the Parp1 protein, as well as Parp2, topoisomerase II alpha, Klf4, Oct4, and Sox2, was substantially downregulated (Fig. 1, panel F, right). Taken together, the differential profiles of Parp1/PARylation activity during reprogramming and tridermal differentiation suggested that Parp1/PARylation may play an important role in the regulation of reprogramming efficiency and the acquisition of pluripotent properties.

### 2. Parp1 and PARylation regulate the efficiency of iPSC reprogramming

We next evaluated whether inhibition of PARylation or knockdown of Parp1 interfere with cell reprogramming. To investigate the role of Parp1 in the early phase of the reprogramming process, we first confirmed the effect of Parp1 knockdown in two MEF-derived iPSC clones 11 days after OSKM transfection by western blotting. In the two reprogrammed clones with Parp1 knocked down, Parp1 expression was almost undetectable at day 11 post-reprogramming (Fig. 2, panel A). The two clones of cells transfected with OSKM and shRNA against Parp1 (OSKM+shParp1) had significantly reduced self-renewal and proliferative capabilities (Fig. 2, panel B), formed smaller colonies, and were less positive for ALP staining (Fig. 2, panel C) compared to cells transfected with scrambled control shRNA (OSKM+ Scramble shRNA). Meanwhile, the resultant ESC markers, including Oct4 and SSEA-1, were significantly inhibited by this Parp1 knockdown (Fig. 2, panel D). To further validate that Parp1 facilitates cell reprogramming, we co-overexpressed Parp1 with either OSKM or OSK in MEFs using a lentiviral transfection system. Western blots confirmed the overexpression of Parp1 at day 11 post-reprogramming (Fig. 2, panel E). We subsequently examined the effect of either Parp1 knockdown or overexpression on the efficiency of iPSC generation at day 21 post-reprogramming. Parp1 overexpression significantly enhanced the reprogramming efficiency in MEFs transfected with OSKM or OSK (Fig 2, panel F, left and middle, respectively). Notably, Parp2 overexpression also enhanced the reprogramming efficiency in MEFs transfected with OSK (Fig. 2, panel F, right), but the effect of Parp2 overexpression was significantly less than that of Parp1 overexpression. Moreover, administration of various PARylation inhibitors consistently led to reduction in the efficiency of iPSC generation induced by OSKM at day 21 post-reprogramming (PJ-34: Fig. 2, panel G, left; ABT-888 and 3-aminobenzamide: data not shown). Parp1 knockdown by a lentivirus-delivered shRNA led to a significant inhibition of the efficiency of iPSC generation (Fig. 2, panel G, middle), and Parp2 knockdown also suppressed iPSC generation at a similar extent at day 21 post-reprogramming (Fig. 2, panel G, right). Taken together, these data indicate that modulating Parp1 and PARylation activity influences the reprogramming efficiency and the pluripotent status of iPSCs, indicating that Parp1 and PARylation are crucial for nuclear reprogramming.

### 3. Replacement of Klf4 or c-Myc with Parp1 in OSKM reprogramming produces iPSCs and generates chimeric animals

c-Myc, a proto-oncogene, is an essential factor for enhancing reprogramming efficiency, but it also increases the risk of tumorigenicity of the reprogrammed somatic cells (Nakagawa et al., 2010). Because Parp1 can increase the efficiency of iPSC generation in the OSK-transfection protocol, we investigated the potential of Parp1 to replace Klf4 and c-Myc. Remarkably, the iPSC-reprogramming efficiency of OS with Parp1 (OSP) was significantly higher than that of OSK, but it was similar to c-Myc cotransfected with OS (OSM; Fig. 3, panel A). We next attempted to investigate the dependence of Parp1-mediated reprogramming and iPSC generation on the cell cycle. Cell cycle analysis indicated that Parp1 overexpression showed no effect in MEFs, compared to parental MEFs or MEFs transfected with a control vector (Fig. 3, panel B). In addition, we observed a shift of the cell cycle to S-phase in MEFs transfected with OSK and OSP at day 11 post-reprogramming (Fig. 3, panel B). This shift was also observed in pluripotent stem cells, including mESCs, Dr. Yamanaka's iPSC clone (miPSCs), and iPSCs generated by transfection of either OSK or OSP (data not shown), as described previously (Fujii-Yamamoto et al., 2005). These data indicate that the Parp1 effect on reprogramming efficiency and iPSC generation is cell cycle-independent. Furthermore, OSP transfection activated the expression of Nanog-GFP during reprogramming in a Nanog-GFP reporter MEF clone (Fig. 3, panel C). The high passages of OSP-reprogrammed iPSCs were stably positive for markers of mouse ESCs, such as ALP activity (Fig. 3, panel D), an ESC-like gene signature (Fig. 3, panel E), stage-specific embryonic antigen (SSEA-1) and Oct4, and protein of stemness factors (Fig. 3, panel F). Bisulfite sequencing showed that the promoters of Oct4 and Nanog in OSP-iPSCs had much a lower methylation status than parental MEFs (Fig. 3, panel G). Importantly, six weeks after transplantation of these iPSCs into the dorsal flanks of nude mice, we observed the formation of teratomas that contained various tissues, including neuronal epithelium (ectoderm), cartilage and keratinocytes (mesoderm), and smooth muscle (mesoderm) (Fig. 3, panel H, upper and lower left). Furthermore, we injected these OSP-iPSCs into blastocysts that were then transplanted into the uteruses of pseudo-pregnant mice. The adult chimeras were confirmed by coat color, demonstrating that OSP-iPSCs were competent to produce adult chimeric mice (Fig. 3, panel H, lower right). These observations indicate that Parp1 overexpression efficiently enhances the reprogramming of mouse somatic cells into iPSCs in the absence of c-Myc or Klf4.

### 4. c-Myc is a direct regulator of Parp1 and PARylation

Given that Parp1 is upregulated during reprogramming, we hypothesized that one or more of the exogenous transcription factors Oct4, Sox2, Klf4, and c-Myc may be the upstream regulators that induce Parp1 expression and PARylation activity. Therefore, we assessed the effects of forced expression of individual or combined Yamanaka's factors on Parp1 expression and PARylation activity in MEFs (Fig. 4, panel A). Five days after gene transfection, forced overexpression of c-Myc alone or transfection of OSM and OSKM resulted in substantial increases in Parp1 protein expression, as well as in PARylation activity in MEFs (Fig. 4, panel A). To further address whether Parp1 is the major downstream effector of c-Myc in the reprogramming process, we knocked down c-Myc and overexpressed Parp1 plus OSK in MEFs. The result suggested that overexpression of Parp1 compensates for c-Myc knockdown and allows efficient reprogramming without c-Myc (Fig. 4, panel B). In addition, c-Myc knockdown significantly blocked ALP activity (data not shown), and suppressed the protein-level of Parp1, Oct4, Sox2, Klf4 and Nanog as well as PARylation activity in iPSCs (Fig. 4, panel C). Taken together, these results indicate that the activation of Parp1 and Parp1 -related PARylation, partly regulated by c-Myc, play a crucial role in facilitating reprogramming and maintaining the pluripotent state of stem cells.

We next determined whether c-Myc regulates Parp1 expression by fusing the Parp1 promoter to a luciferase reporter plasmid and co-expressing the reporter with c-Myc. Three putative c-Myc-binding sites were identified in the proximal promoter region (-2000 to -100 base pairs) of Parp1 and deletion constructs were cloned in the luciferase reporter plasmid (Fig. 4, panel D). Cotransfection experiments showed that c-Myc activated the transcriptional activity of the Parp1 promoter containing three (-2000) or two proximal (-1100) c-Myc binding sites. In contrast, the Parp1 promoter deletion mutants lacking c-Myc-C1 and c-Myc-C2 (-600) suppressed c-Myc-activated Parp1 transcription (Fig. 4, panel E), indicating that C2 is an important site responding to c-MyC activity. Consistently, the Parp1 promoter construct without all three c-Myc binding sites (-125) or with point mutations in C2 (c-Myc-C2 mt) could not be stimulated by c-Myc. To investigate whether c-Myc can directly bind to the C2 c-Myc binding site in the promoter region of Parp1, chromatin immunoprecipitation (ChIP) assays were performed using C1, C2, and C3 primer sets (Fig. 4, panel F). The result showed that the endogenous c-Myc indeed only bound to the C2, but not C1 or C3, position of the Parp1 promoter. As a positive control, c-Myc bound to its reported target, cyclin D2 promoter. Panel Gof Fig. 4 shows the result of panel Fof Fig. 4 ChIP with quantitative PCR (qChIP). These data strongly suggest that the Parp1 promoter region containing C2 was required for maximal activity of Parp1 in iPSCs. Together we demonstrated that c-Myc is a direct regulator of Parp1 and PARylation.

### 5. Identification of PARylated targets and expression levels of Parp1/PARylation-associated proteins in pluripotent and differentiated states

PARylation was previously considered the major catalytic function of Parp1; we therefore attempted to identify the proteins that are involved in Parp1-mediated PARylation in pluripotent stem cells. We used poly(ADP-ribose) affinity resin to pull down the PARylated proteins in iPSCs and MEFs. The PARylated proteins indentified by LC-MS/MS in iPSCs are listed in Table 1. Among these candidate proteins, Parp1, Chd1L, DNA ligase III, Ssrp1, Xrcc6, and Parp2 were identified by LC-MS/MS as having more than 3 peptides per protein. To confirm these results, we used western blotting with specific antibodies to detect the expression of these candidate proteins. Compared to the input (total lysate) column, there was no detectable signal in the negative control column (resin with a mutated affinity domain), and specific signals from these six proteins were only observed in iPSCs following PAR affinity resin purification (Fig. 5, panel A). We attempted to further evaluate the expression profiles of these candidate proteins in the reprogramming process and pluripotent stem cells. First, we found that the total protein levels of Chd1L, DNA ligase III, Ssrp1, and Xrcc-6 were modest in MEF lysates, and gradually increased in the reprogramming process (Fig. 5, panel B). The total protein expression of Chd1L was not correlated with pluripotency as strongly as it was with the reprogramming state (Fig. 5, panel B). In contrast, when PAR affinity resin was used to pull down these candidate proteins from total cell extracts, all candidate proteins were gradually PARylated during the reprogramming process, and the maximal PARylation of these proteins was found in iPSCs, Yamanaka's miPSC clone (miPSC), and mESCs, but not in MEFs (Fig. 5, panel C). Notably, as detected by the pull-down assays, Parp2 levels in the reprogramming D6 and D12 were not significantly changed, compared to Parp1 (Fig. 5, panels B and C). Co-immunoprecipitation further confirmed that Parp2, Chd1L, DNA ligase III, Xrcc-6, and Ssrpl interact with Parp1 to form a complex (Fig. 5, panel D). To explore whether differentiation affects the expression of PARylated proteins, control (total input of cell lysate; Fig. 5, panel E) and PAR-resin pull-downs (Fig. 5, panel F) from iPSC-derived embryonic bodies (EBs) were compared. In the total input, there were no significant changes of the six proteins on day 3, day 6, and day 9 after ED differentiation (Fig. 5, panel E). The expression of PARylated Parp1, Chd1L, DNA ligase III, Ssrp1, Xrcc-6, and Parp2 was decreased during the differentiation process of iPSC-derived EBs (Fig. 5, panel F). Notably, the expression levels of these six PARylated proteins were significantly downregulated on day 9 after EB differentiation (Fig. 5, panel F). These data suggest that PARylated levels of Chd1L, DNA ligase III, SSrp1, Xrcc-6/Ku-70 and Parp2 increased during reprogramming and were high in pluripotent state of cells, but decreased during the differentiation process.

In addition, we further explored the roles of Parp1 in posttranslational modification to gain additional insights into the functional consequences of differential patterns of PARylated proteins in pluripotent stem cells. Using gene network analysis with the IPA software package to construct network modules, we found that Parp1 may be a key factor regulating the pathways related to DNA repair, chromatin modification, the polycomb complex, and histone modification. Remarkably, the bioinformatic analysis revealed that Parp1-PARylated proteins interacted significantly with Oct4, Nanog, c-Myc, Klf4, CTNNB1, WDR5, SUZ12, EZH2, DNMT3A/B, and JARID2 in the core network of nuclear reprogramming and pluripotent status (Fig. 6). In addition, we further explored the roles of Parp1 in posttranslational modification to gain additional insights into the functional consequences of differential patterns of PARylated proteins in pluripotent stem cells. Using gene network analysis with the IPA software package to construct network modules, we found that Parp1 may be a key factor regulating the pathways related to DNA repair, chromatin modification, the polycomb complex, and histone modification. Remarkably, the bioinformatic analysis revealed that Parp1-PARylated proteins interacted significantly with Oct4, Nanog, c-Myc, Klf4, CTNNB1, WDR5, SUZ12, EZH2, DNMT3A/B, and JARID2 in the core network of nuclear reprogramming and pluripotent status (Fig. 6).

In conclusion,using a proteomic approach, we compared the nuclear protein expression profiles among MEFs, ESCs, and iPSCs, and we identified Parp1 as a pivotal regulator of nuclear reprogramming and pluripotency. Our data demonstrated that the expression of Parp1 and PARylation increased during reprogramming and decreased upon differentiation. Parp1 replaced Klf4 or c-Myc in promoting iPSC production and generating chimeric mice with Oct4/Sox2-transfected cells (Fig. 3). We further showed that c-Myc directly binds to the Parp1 promoter to enhance its expression, resulting in increased PARylation activity. The reduced reprogramming efficiency of MEFs transfected with OSK plus RNAi against c-Myc was rescued by ectopic Parp1 (Fig. 4). Finally, we demonstrated that Parp1 interacted with several DNA repair- and chromatin remodeling-associated proteins, which were highly expressed and PARylated in reprogrammed and pluripotent cells (Fig. 5). These data indicate that the activation of Parp1 and PARylation, partly through endogenous c-Myc, effectively promotes nuclear reprogramming and the maintenance of pluripotency.

### Examples - (II)

### I. Ventilator-Induced Lung Injury Model

Acute lung injury (ALI) and acute respiratory distress syndrome (ARDS) are disordersof acute respiratory failure and manifest as non-cardiogenic pulmonary edema, respiratorydistress and hypoxemia. High tidal volume-induced mechanical ventilation in patients hasbeen shown to increase the risk of pathologic overdistention in the lungs, elicit the productionof inflammatory mediators, recruit inflammatory cells, and eventually induce a type of ALI, termed ventilator-induced lung injury (VILI). Recently, the treatment efficacy ofmesenchymal stem cells (MSCs) to modulate inflammatory responses has been demonstratedin sepsis-induced ALI (Mei SH et al. Am J Respir Crit Care Med2010;182:1047-1057; and Chien MH et al. CritCare Med 2012;40:1245-1253). Another study further indicated that MSC therapy enhancedlung repair in VILI through a keratinocyte growth factor-dependent paracrine mechanism (Curley GF et al. Thorax 2012;67:496-501). However, the stem cell therapy-based biomolecular mechanisms that improved ALI or VILI remain unknown.

### 1. Materials and Methods

### 1.1 iPSC generation by transducing Oct4/Sox2/Klf4

Mouse embryonic fibroblasts (MEFs) were reprogrammed into iPSCs by ectopicallytransfection of Oct4/Sox2/Klf4 (OSK) as previously described (Li HY et al. Biomaterials 2011;32:5994-6005). Undifferentiated iPSCswere cultured on inactivated MEF, and formed colonies very similar to ESCs.These iPSC clones were positive for alkaline phosphatase (AP) and SSEA-1, detected by AP and immunofluorescent staining, respectively. We investigated the pluripotencyof these iPSCs using embryoid body formation and various differentiationprotocols. The iPSCs were able to differentiate into chondrocyte-like cells, osteocyte-like cells, andhepatocyte-like cells and neuronal-lineaged cells (data not shown).

### 1.2 Experimental animals

Male C57BL/6 weighing between 20 and 25 g, aged between 6 and 8 weeks, were obtained from Jackson Laboratories (Bar Harbor, ME) and National Laboratory Animal Center (Taipei, Taiwan). Mouse model of VILI was established as previously described (Li LF et al. Respir Res 2011;12:90). Animals were randomly distributed into seven groups in each experiment: group 1, control, nonventilated mice with phosphate-buffered saline (PBS); group 2, control, nonventilated mice with induced pluripotent cells (iPS) cells; group 3, V_{T} 6 ml/kg mice with PBS; group 4, V_{T} 30 ml/kg mice with PBS; group 5, V_{T} 30 ml/kg mice with mouse embryonic fibroblast (MEF); group 6, V_{T} 30 ml/kg mice with iPSCs; group 7, V_{T}30 ml/kg mice with conditioned medium of iPSCs; (n = 5 each for Evans blue dye (EBD) assay, bronchoalveolar lavage (BAL) total protein, lung water, neutrophils, myeloperoxidase (MPO), HMGB1 and PAI-1 mRNA, immunohistochemistry (IHC), terminal deoxynucleotidyl transferase-mediated dUTP-biotin nick end-labeling (TUNEL),hematoxylin and eosin (H&E) stain; n = 3 for electron microscopy).

### 1.3 Reverse transcription-polymerase chain reaction

For isolating total RNA, the lung tissues were homogenized in TRIzol reagents(Invitrogen Corporation, Carlsbad, CA) according to the manufacturer's instructions. Total RNA (1 µg) was reverse transcribed by using a GeneAmp PCR system 9600 (PerkinElmer,Life Sciences, Inc., Boston, MA), as previously described (Li LF et al. Respir Res 2011;12:90).

### 1.4 Evaluation of lung injury

Analysis of lung water, Evans blue dye analysis, histopathologic grading of VILI,cell counts andmyeloperoxidase assay were described in Chien MH et al(CritCare Med 2012;40:1245-1253).

For the measurement of PAI-1 and HMGB1, the lungs were lavaged via tracheostomy with a 20-gauge angiocatheter (sham instillation) 3 times with 0.6 ml of 0.9% normal salineat the end of the study period. The effluents were pooled and centrifuged at 2,000 rpm for 10 min. Supernatants were frozen at -80°C for further analysis of the cytokine. PAI-1 with a lower detection limit of 0.02 ng/ml and HMGB1 with a lower detection limit of 1 ng/ml were measured in BAL fluid using a commercially available immunoassay kit containing antibodies that were cross-reactive with rat and mouse PAI-1 (Molecular Innovations, Inc., Southfield, MI) and HMGB1 (Shino-Test corporation, Kanagawa, Japan). Each sample was run in duplicate according to the manufacturer's instructions.

For transmission electron microscopy,the lungs were fixed in 3% glutaraldehyde in 0.1 M cacodylate buffer (pH 7.4) for 1 h at 4 °C. The lungs were then postfixed in 1% osmium tetroxide (pH 7.4), dehydrated in a graded series of ethanol, and embedded in EPON-812. Thin sections (70 nm) were cut, stained with uranyl acetate and lead citrate, and examined on a Hitachi H-7500 EM transmission electron microscope (Hitachi, Ltd., Tokyo, Japan).

### 1.5 Statistical evaluation

The data of lung water, BAL total protein, EBD assay, PAI-1 and HMGB1, MPO, and histopathologic assay were analyzed using Statview 5.0 (Abascus Concepts Inc. Cary, NC; SAS Institute, Inc.). ANOVA was used to assess the statistical significance of the differences, followed by multiple comparisons with a Scheffe's test, and a P value < 0.05 was considered statistically significant.

### 2. Results

### 2.1 IPSCs or iPSC-CM attenuated high tidal volume-induced VILI

We employed high tidal volume (V_{T}30 ml; denoted as V_{T}30)ventilation with ambient air for 4 hours to induce VILI in male C57BL/6 mice and examined the treatment effects of intravenously delivered iPSCs or iPSC-conditioned medium(iPSC-CM). Gross pathologic findings indicated that the animal lungs injured by mechanicalventilation at V_{T}30, but not at a low tidal volume (V_{T}6 ml, denoted as V_{T}6), displayed apattern of hemorrhaging, severe congestion and enlargement due to edema (Fig.7, panel A). AV_{T}30 also increased lung Evans blue dye (EBD) content, bronchoalveolar lavage (BAL) totalprotein, and the wet-to-dry ratio, indicating capillary leakage. However, a V_{T}6 showed noeffect on these parameters when compared with non-ventilated mice (Fig.7, panels B-D). The macroscopic lung congestion and elevation of capillary permeability induced by a V_{T}30 was not affected by MEF treatment, but was substantially suppressed by treatment with eitheriPSCs or iPSC-CM (Fig. 7, panels B-D). Furthermore, the PaO₂/FiO₂ ratio, an index of gasexchange, was significantly deteriorated with a V_{T}30 when compared with non-ventilatedmice or mice receiving a V_{T}6 (Fig.7, panel E). Remarkably, the decreases in oxygenation with a V_{T}30 were significantly improved by the administration iPSCs or iPS-CM. Therefore, thesedata suggest that iPSCs or iPSC-CM improve microvascular leakage, lung edema, total lunginjury, and help to recover respiratory functions in a VILI model induced by a V_{T}30.

### 2.2 IPSCs or iPSC-CM suppressed the VILI-associated inflammatory response

We next examined if iPSCs or iPSC-CM led to structural recovery in this VILI model. Histological examination revealed that a V_{T}30 led to alveolar congestion, hemorrhaging,thickening of the alveolar wall, and neutrophil infiltration, which were largely rescued by the administration of iPSCs or iPSC-CM (Fig.8, panel A). The lung injury score quantificationconfirmed the V_{T}30-induced severe damage and the therapeutic potential of iPSCs andiPSC-CM (Fig.8, panel B). The neutrophil counts and myeloperoxidase (MPO) assay revealedthat neutrophils migrated into the injured lung sites in mice after mechanical ventilation at V_{T}30 when compared with non-ventilated mice or mice receiving a V_{T}6 (Fig.8, panels C and D).Meanwhile, the HMGB1 and PAI-1 protein levels were elevated in response to V_{T}30 treatment (Fig.8, panels E and F), indicating an upregulation of chemoattractants for neutrophils inthis model. Significantly, iPSC or iPSC-CM ameliorated neutrophil migration and HMGB1 and PAI-1 protein elevation (Fig.8, panels C-F). The inhibitory effects of iPSC oriPSC-CM on lung injury scores and neutrophil migrationwere dose-dependent, and maximum inhibition was observed in high tidal volume-inducedALI receiving 5×10⁵ iPSC/kg or the corresponding iPSC-CM (data not shown). These data demonstrate that both iPSCs and iPSC-CM attenuate neutrophilinfiltration and inflammatory responses in high tidal volume-induced ALI.

### 2.3 Ultramicrostructural restoration by iPSC-CM

Transmission electron microscopy (TEM) showed that administration of V_{T}30, but notV_{T}6, led to acute injury of the airway ultramicrostructure in the recipients of MEF or PBS(Fig. 9, panel A). Administration of iPSCs or iPSC-CM consistently restored the airwayultramicrostructure in the recipients (Fig.9, panel A).Based upon the observations of the restorative effect ofiPSCs and iPSC-CM on VILI (Fig.7 and 8), the iPSCs exerted their protective functions in apredominantly paracrine manners. In addition to the effect on the respiratory parameters,neutrophil infiltration and chemoattractant expression, we investigated the effect of V_{T}30 andiPSC-CM administration on the expression of macrophage inflammatory protein-2 (MIP2),nitrate/nitrite, malondialdehyde (MDA) and total glutathione (GSH) from lung tissues inrecipients. Along the induction of VILI, V_{T}30 recruited the production of MIP2 chemoattractant and nitrate/nitrite, MDAcontent and decreased GSH production (Fig.9, panels B-E). Significantly,iPSC-CM administration effectively inhibited the upregulation of MIP2, nitrate/nitrite, and the production of MDA, but elevated GSH production in recipients (Fig.9, panels B-E). In addition, we found in a cytokine array that several cytokines, including uPA and TIMP-4, were also secreted by iPSCsinto the conditioned medium (Fig.9, panel F).

### 2.4 IP-10 is involved in the reparative response of iPSC-CM in VILI

IP-10, monokine induced by IFN-γ (MIG) and the IFN-γ inducible T-cell chemoattractant (iTAC) are three chemokines that bind to acommon receptor, CXCR3. These three chemokines can be induced by INF-y.Among these chemokines, IP-10 has exhibited protective ability against hepatitis,pulmonary fibrosis, and myocardial infarction and has been involved in tissuerepair and remodeling. Accordingly, we investigated whether IP-10 was involved in the reparative effect of iPSC-CM in the V_{T}30-induced VILI model. Quantitative RT-PCRindicated that V_{T}30 mildly increased the expression of IP-10 and MIG (Fig.10, panel A), butshowed no effect on CXCR3 expression (data not shown). The transplantation of iPSCs largely increased the expression of IP-10 and MIG,while the administration of iPSC-CM alone moderately increased their levels (Fig.10, panel A).ELISA data revealed that iPSCs andiPSC-CM stimulated IP-10 secretion in a pattern similar to its mRNA level (Fig.10, panel B).To examine the contribution of IP-10 in the reparative effect of iPSC-CM, we evaluated the effect of IP-10 neutralization by administration of IP-10 neutralizing antibody (IP-10 nAb). IP-10 nAb alone significantlyimpaired structural changes, lung injury scores, neutrophil infiltration, and the PaO2/FiO2 ratio in V_{T}30-treated mice. IP-10 nAb also substantially block the reparative effectproduced by iPSC-CM on these parameters (Fig.10, panels C-E). Taken together, these findings demonstrate that IP- 10 serves a pivotal role and is involved in the reparative effect of iPSC-CM on airway structuraldamage and oxygenation ability in VILI.

### II. Bleomycin-Induced Lung Inflammation Model

Pulmonary fibrosismanifests clinically during acute respiratory distress syndrome (ARDS), which has a mortality rate of upto 40%, and during idiopathic pulmonary fibrosis (IPF), which is characterized by aggressive fibroticprogress and represents a major burden to global health. Bleomycin has been used to model fibroticlung injury in animal studies, as the characteristics of bleomycin-induced lung damage in animals includeacute inflammatory injury of the alveolar epithelium followed by reversible fibrosis, which overlaps with the symptoms of ARDS and IPF. The extent of fibrosis is also proportional to the severity of the initialinjury. Intra-trachealadministration of bleomycin requires only a single dose to result in injury and fibrosis, with fibrosis starting after 14 days and maximal responses occurring after 21-28 days. Previousstudies have suggested that the early reduction of inflammation may result in the attenuation ofdownstream events leading to collagen deposition (Moodley Y et al. Am J Pathol 2009; 175:303-313). Developing an effective strategy for amelioratingpulmonary damage during the early phases of pulmonary fibrosis pathogenesis is therefore a top priority.

### 1. Materials and Methods

### 1.1 iPSC generation, iPSC culture, and preparation of the Conditioned Medium

Mouse iPSCs, which express a gene signature similar to that of ESCs (i.e., the expression of Oct4,Sox2, Nanog, Klf-4, Fbx5, Eras, Dppa5a, and Rex1 and a pluripotent status), were established by introducing three genes (Oct4/Sox2/Klf4) not including c-Myc. Briefly, murine iPSCs were generatedfrom mouse embryonic fibroblasts (MEFs) derived from 13.5-day-old embryos of C57/B6 mice. TheiPSCs were reprogrammed via the transduction of pMX-based retroviral vectors encoding threetranscription factors, Oct-4, Sox2, and Klf4, according to the protocol described in previous studies with minor modifications(Takahashi K et al. Cell 2006; 126:663-676). Plat-E packing cells were incubated overnight at a density of 3.6x10⁶ cells per 100-mm dish. The next day, pMX-based retroviral vectors encoding mouse complementary DNAs wereintroduced into the Plat-E cells using the Fugene 6 transfection reagent (Roche Applied Science,Indianapolis, IN). Forty-eight hours after transfection, virus-containing supernatants were collected for target cell infection. In preparation for viral infection, 8x10⁵ MEFs were seeded per well into 6-wellplates one day before transduction. The virus-containing supernatants were filtered through a 0.45-µm filter and supplemented with 4 µg/ml polybrene (Sigma-Aldrich, St. Louis, MO). Equal amounts of supernatants containing each of the three retroviruses were mixed, transferred to the fibroblast dish, and incubated overnight. After infection, the cells were re-plated in fresh medium. Six days after transduction,the cells were passaged on an SNL feeder layer and cultured using mouse ESC culture medium. Colonieswere selected for 2 to 3 weeks.

Formouse iPSC culture, undifferentiated iPSCs were routinely cultured and expanded on mitotically inactivated MEFs(50,000 cells/cm²) in six-well culture plates (BD Technology, Franklin Lakes, NJ) in the presence of 0.3% leukemia inhibitory factor (LIF) in an iPS medium consisting of Dulbecco's modified Eagle'smedium (DMEM; Sigma-Aldrich) supplemented with 15% fetal bovine serum (FBS; Invitrogen, Carlsbad,CA), 100 mM minimal essential medium nonessential amino acids (Sigma-Aldrich), 0.55 mM2-mercaptoethanol (Gibco, Gaithersburg, MD), and antibiotics (Invitrogen). Every 3-4 days, colonieswere detached with 0.2% collagenase IV (Invitrogen), dissociated into single cells with 0.025% trypsin(Sigma-Aldrich) and 0.1% chicken serum (Invitrogen) in phosphate-buffered saline (PBS), and re-platedonto MEFs.

For iPSC conditioned medium, mouse iPSCs were placed at 20,000 cells per cm² and incubated in a 10-ml volume of serum-freebasal medium (DMEM-high glucose (Gibco) with a 100 mM concentration of nonessential amino acids(Gibco), 0.3% LIF, and 1% penicillin-streptomycin) in a 10-cm dish (Corning Incorporated) for 48 h.Then, the trypsinized iPSCs in whole culture medium were collected and centrifuged for 10 min at 1500rpm to obtain the supernatant to be used as the conditioned medium in subsequent in vivo experiments.

### 1.2 Experimental animals

C57BL/6J mice (from the National Laboratory Animal Center, Taipei, Taiwan) that were 8-10 weeks of age were maintained on a 12-h light-dark cycle and were provided free access to food and water. TheEthical Committee for the Use of Laboratory Animals of Taipei Veterans General Hospital approved the experimental procedures and protocols, which also complied with the "Guide for the Care and Use ofLaboratory Animals".

C57BL/6J mice were intra-tracheally injected with 1.5 U/kg bleomycin sulphate (Merk, Darmstadt,Germany) in 50 µl PBS under light anesthesia to induce pulmonary fibrosis. In the designatedexperiments, mice received either iPSCs (2×106 cells in 200µl of PBS; iPSC-treated mice) or PBS 200 µl(PBS-treated mice) via tail vein injection 24h after the induction of lung injury. Cell administration 24hours after lung injury was chosen to optimize cell incorporation into the lungs during early inflammation. For neutralizing antibody study, mouse was given two doses (0.5µg/dose, i.p.) of anti-IP-10 antibodies(Abcam, ab9938, Cambridge, UK) at 4 h before and 4 h after injury.

Further experiments were performed in which non-reprogrammed MEFs (2×10⁶ cells in 200µl ofPBS; MEF-treated mice) and the conditioned medium from iPSCs (200 µl of iPSC-CM) were injectedinto the tail vein 24 hours after the induction of bleomycin-induced lung injury as control cell therapy andparacrine therapy, respectively. The animals were sacrificed after 3, 7, 14, or 21 days post-bleomycininjection because these time points represent the phases of maximal inflammation (3 and 7 days) and fibrosis (14 and 21 days). Samples were collected from each mouse for the assessment of lung injury,which was performed according to the hydroxyproline assay, pulmonary physiology, histology,immunohistochemistry, and cytokine and myeloperoxidase (MPO) analysis.

### 1.3 Histology, Ashcroft Lung Fibrosis Score and Morphological Analysis

Lungs from each group of animals were excised at 7, 14, and 21 days after bleomycin-induced lunginjury. The lung tissues were fixed in 4% paraformaldehyde, dehydrated using a graded ethanol series,embedded in paraffin blocks, cut into 3-µm sections, and stained with hematoxylin and eosin (H&E)using standard histologic techniques. Blind histopathologic evaluation of the severity of pulmonaryfibrosis was performed by two separate investigators using the Ashcroft scoring method (Ashcroft T et al. J Clin Pathol 1988; 41:467-470). Lung fibrosiswas scored on a scale of 0 to 8 according to the following criteria: grade 0, normal lung; grade 1-2, minimal fibrous thickening of alveolar or bronchiolar wall; grade 3-4, moderate thickening of walls without obvious damage to lung architecture; grade 5-6, increased fibrosis with definite damage to lungstructure; and grade 7-8, severe distortion of structure and large fibrous areas. Following the examinationof 30 randomly chosen regions in each sample at 100x magnification, the mean score of all the fields wastaken as the fibrosis score for each sample.The destruction of alveolar walls was quantified according to the destructive index value, which wasmeasured using a previously described technique (Saetta M et al. Am Rev Respir Dis 1985; 131:764-769). Airspace size was quantified by measuring the meanlinear intercept (Lm) using a modified Thurlbeck method (Thurlbeck WM. Am Rev Respir Dis 1967; 95:752-764). Briefly, 30 fields at 200x magnification wererandomly observed on two slides from each mouse, and point counting in each field was performed. Then, the total distance divided by the number of alveolar intercepts provided the value for Lm.

### 1.4 Immunohistochemistry

Immunohistochemical staining was performed using primary antibodies targeting Ly6C (Santa CruzBiotechnology, Santa Cruz, CA), collagen I (Abcam, Cambridge, UK), and alpha-smooth muscle actin(α-SMA) (Biomeda, Foster City, CA). Briefly, paraffin sections of the lungs were de-waxed and rehydrated. Lung sections were subjected to antigen retrieval and blocked with a peroxidase-blockingreagent. Sections were incubated with primary antibody overnight at 4°C. After washing, the lungsections were incubated with a SuperSensitiveTM system horseradish peroxidase-labeled polymer(BioGeneX, San Ramon, CA) for 1 hour at room temperature. The sections were then visualized with3,3'-diaminobenzidine tetrahydrochloride (BioGeneX, San Ramon, CA) and counterstained with hematoxylin. To quantitatively analyze the IHC intensity of the scratched area, the percentage of IHCsignal per photographed field was analyzed using Image Pro Plus software (Media Cybernetics, Inc.,Silver Spring, MD). The mean value of 15 randomly selected fields was calculated.

### 1.5 Hydroxyproline Assay

Homogenates from frozen lung tissues were incubated on ice in trichloroacetic acid and thenbaked in 12N HCl. Aliquots of the samples were reconstituted in distilled water and then incubated inl.4% chloramine T in 10% isopropanol and 0.5 M sodium acetate for 20 min. Erlich's solution was added,and the samples were incubated at 65°C for 15 minutes. Absorbance at 550 nm was measured, and the amount of hydroxyproline was determined according to comparisons against a standard curve.

### 1.6Cytokine ELISA Array

Lung tissues for ELISA were homogenized in PBS with protease inhibitors containing 10 µg/mLAprotinin, 10 µg/mL Leupeptin, and 10 µg/mL Pepstatin. Triton X-100 was added to a final concentrationof 1%. Samples were frozen at ≤ -70°C, thawed, and centrifuged at 10,000 g for 5 min to remove cellulardebris. Quantitation of sample protein concentrations in the supernatants was performed using a totalprotein assay. Cytokine levels were determined according to the manufacturer's instructions provided with commercial ELISA kits (R&D Systems, Minneapolis, MN). Cytokine array data on developed X-rayfilm was quantified by scanning the film with an ImageScanner III (GE Healthcare) and analyzing the array image file using ImageQuant software (GE Healthcare).

### 2. Results

### 2.1Effects of iPSCs and iPSC-conditioned medium on the Histopathology of Bleomycin-InducedLung Injury

The intra-tracheal injection of bleomycin resulted in the previously documented sequence of eventsleading to lung injury. The early phase of injury was characterized by neutrophilic alveolitis, whereas the late phase wascharacterized by patchy areas of fibrosis,asevidenced by the histopathological examination of bleomycin-injuredlungs. The intravenous delivery of iPSCs or iPSC-CM, but notMEFs, reduced the number of infiltrative neutrophils and injury areas observed in bleomycin-injuredlungs at day 7 post-bleomycin treatment. In addition, bleomycin-induced interstitial thickening, inflammation, and distortion of lung architecture were attenuated following the administration ofiPSCs or iPSC-CM at days 14 and 21 post-bleomycin treatment. The bleomycin-treatedrecipients of iPSCs also demonstrated significantly lower Ashcroft lung fibrosis scores than thoseof MEFs or PBS alone (Fig.11,panel A). Restoration of bleomycin-impaired lung structure following treatment with iPSCs was also observed according to measurements of the destructive index (Fig.11,panel B). Therestorative effect of iPSC-CM on Ashcroft lung fibrosis scores and the destructive index was slightly lessthan that of iPSCs. The alveolar mean linear intercept (Lm), a morphometric parameter of averagealveolus size, also recovered to near-normal levels following the delivery of iPSCs or iPSC-CM(Fig.11,panel C).

### 2.2 Both iPSCs and iPSC-CM Attenuate the Intensity of Bleomycin-Induced Pulmonary Fibrosis

Pulmonary fibrosis is characterized by the accumulation of fibrillar collagens like type 1 collagen. Immuno-staining for collagen-1 demonstrated thick bands of fibrosis in PBS-treated mice, while the lungs of bleomycin-challenged recipients treated with either iPSCs or iPSC-CM were significantlyprotected from bleomycin-induced fibrosis (Fig. 12, panel A). The accumulation of myofibroblasts,which are considered important fibrogenic effector cells, was also examined. As indicated by the staining of lung sections for α-SMA at 14 and 21 days post-bleomycin treatment, recipients of iPSCs oriPSC-CM mice exhibited a significant decrease in myofibroblast accumulation in comparison toPBS-treated mice (Fig.12, panel B). Lung collagen content was further assessed by measuringhydroxyproline content. Values in the iPSC-treated group and iPSC-CM-treated group were significantlylower than those in the PBS-treated group at 14 days and 21 days, indicating that bleomycin-inducedcollagen synthesis was significantly suppressed in mice treated with iPSCs or iPSC-CM (Fig. 12, panel C).

### 2.3 IPSCs Increased Production of Anti-Fibrotic Chemokine IP-10 in Injured Lungs

Because we observed reduced inflammation and fibrosis following bleomycin-induced lung injury asa result of treatment with iPSCs and iPSC-CM, we next sought to investigate the potentialmechanisms responsible for this effect. As the transition from inflammation to fibrosis occurs at an earlyphase during pathogenesis, we hypothesized that cytokine profiling would reflect the influence ofcytokines on subsequent collagen deposition. The effect of iPSCs and iPSC-CM on lung cytokineand chemokine levels was evaluated using a cytokine array assay (Fig.13,panel A). Following treatment withiPSCs or iPSC-CM, there was a reduction in the expression of cytokines and chemokines knownto mediate inflammation (i.e., IL-1, IL-2, IL-10, TNF-α, and MCP-1) and fibrosis (i.e., INF-γ andMCP-5).In addition to demonstrating reduced levels of severalcytokines/chemokines capable of mediating inflammation and fibrosis, the ELISA data also showed thatboth iPSCs and iPSC-CM treatment stimulated the production of IP-10 (Fig.13,panel A). Real-time PCRfurther indicated that administration of iPSCs or iPSC-CM significantly increased the mRNAexpression of IP-10 at 3 days post-bleomycin injury (Fig. 13,panel B). In addition, these treatments reduced INF-γ mRNA expression but showed no effect on MIG, iTAC, or CXCR3 mRNA expression (Fig. 13,panels C-E).

### 2.4 IPSCs and iPSC-CM rescued the survival of bleomycin-treated recipient in a celldose-dependent manner

To verify the protective effect of iPSCs and iPSC-CM against bleomycin-induced pulmonaryfibrosis, we also evaluated and compared the treatment efficacy of iPSCs to that of iPSC-CMregarding recipient survival in response to bleomycin challenge. As shown in Fig.14,panel A, mice were firstintra-tracheally injected with 3.0 U/kg bleomycin sulphate, a dose sufficient to induce recipient lethalit.Notably, the intravenous delivery of iPSCs led to a dose-dependent improvement in recipientsurvival, and the maximal protective effect of iPSCs was observed at a dose of 2 × 10⁶ cells. Treatment with iPSC-CM (collected from iPSC culture at the indicated cell dosages) similarly improvedrecipient survival in a cell dose-dependent manner (Fig. 14,panel B). Collectively and based on the observedprotective effects of iPSCs and iPSC-CM on pulmonary fibrosis and recipient survival, theseresults suggest that iPSCs exerted their protective effect in a predominantly paracrine manner.

### 2.5 IP-10 as the major contributor to the iPSC-CM-mediated reparative response

To elucidate whether IP-10 plays a crucial role in the reparative effect of iPSC-CM treatment, weevaluated the effect of neutralizing IP-10 by administration of IP-10-neutralizing antibody (IP-10 nAb) attwo distinct doses (i.e. 05µg/dose and 0.3µg/dose). In a cell dose-dependent manner, IP-10 neutralization reduced the survival rate of bleomycin-injected mice that received iPSC-CM treatment (Fig. 15,panel A), or iPSCtransplantation (data not shown). We further demonstrated that IP-10 nAb administration largelyblocked the effects of iPSC-CM on collagen deposition (positive staining for collagen-1; Fig.15,panel B),myofibroblast accumulation (positive staining for α-SMA; Fig.15, panel C), inflammatory cell accumulation(positive staining for Ly6C; Fig.15,panel D), and pulmonary fibrosis (Fig. 15,panel E). Administration of IP-10 nAb alsodeteriorated the reparative effect of iPSCs, identical to the observations in that of iPSC-CM (datanot shown). Taken together, these findings demonstrate that IP-10 is the major contributor to the iPSC and iPSC-CM-mediated inhibition of collagen deposition and inflammation in pulmonaryfibrosis.

### III. CCl4--Induced Acute Liver Injury Model

Liver diseases and injuries are important medical problem worldwide. Liver transplantation iscurrently the most efficient therapy for liver failure and end-stage liver disease. However, it islimited by the scarcity of donor, expensive medical cost, surgical risk and requiring life-longimmunosuppressant agents. The development and application of hepatocytes transplantation hasbeen attempted to treat different forms of liver diseases. It has minimal invasive procedures and fewer surgical complications compared to the orthotopic liver transplantation. Stem celltransplantation has also gained considerable attention recently. Stem cells have the potential tosupportive tissue regeneration and to generates large amounts of donor cells ready for transplant (Kakinuma S et al. J Gastroenterol 44: 167-172; Navarro-Alvarez N et al. Curr Stem Cell Res Ther 2009;4: 141-146; Yoshimi Aet al. BiolBlood Marrow Transplant 2009;15: 389-390; and Si-Tayeb K et al. Hepatology (Baltimore,Md) 2010;51: 297-305)

### 1. Materials and Methods

### 1.1 Cell cultures studies

Mouse germline-competent iPS cells were provided by Kyoto University (Dr. Shinya Yamanaka)and RIKEN BRC, Japan (Takahashi K et al.(2006) Cell 126:663-676). IPS cells were cultured as previously described (Okita K et al. (2007) Nature 448: 313-317.9). The iPS cells weresuccessfully induced to differentiate into hepatocyte-like (iHL) cells with functions resemblingprimary hepatocytes.For recombinant IP-10 (rIP-10, Peprotech, NC) study,hepatocytes were seeded on 24-well plates at the same density. The rIP-10 (0.5ng or 5ng) wasgiven at 4 h post-injury. The viability of hepatocytes was evaluated at 24 h by methyl thiazoltetrazolium (MTT, Sigma) assay.

### 1.2 Experimental animals

Mice (C57/B6, 8 to 10 weeks) were housed in cage and allowed free access to food and water. Mouse was given carbon tetrachloride (CCl4, Sigma) in mineral oil (0.35 µl/g, single dose, i.p.) to induce liver injury. At 4 h post-injury, mice were randomized to receive vehicle (PBS), iPS cells or iHL (2x10⁶ cells/in 100µl PBS) infusions via tail veins. For the time points study, mouse received blood withdrawal (∼100µl) from facial veins at scheduled time for liver biochemistry. When mouse wassacrificed, blood was drawn from the heart and the liver was harvested and prepared for subsequent experiments including histochemistry, cytokine assay, protein and gene expression analysis. For neutralizing antibody study, mouse was given two doses (0.5µg/dose, i.p.) of anti-IP-10 antibodies (Abcam, ab9938, Cambridge, UK) at 4 h before and 4 h after injury. For 72 h survival study, mice received repetitive CCl4 injury at 0, 24 and 48 h. The iPS cells (2x10⁶ cells/in 100µl PBS) or recombinant IP10 (rIP10, 0.5ng) were given once at 4 h and the mortality rate of mice was observed until 72 hpost-injury. All animals received humane care according to the Guide for the Care and Use of Laboratory Animals prepared by the National Academy of Sciences (NIH publication no. 86-23,revised 1985) and approved by the Institutional Animal Care and Use Committee (IACUC) ofTaipei Veterans General Hospital (VGH99-173). All experiments adhered to the AmericanPhysiological Society Guiding Principles for the Care and Use of Laboratory Animals.

### 1.3 Histological quantification of liver injury

The paraffin sections of livers were stained by H.E. and photo-taken under microscopy at 40xmagnification to evaluate the degree of injury. Necrotic area were determined by measuring fiveindependent fields per liver using a computerized morphometry system (MicroCam, M&T OPTICS,Taiwan) and expressed as percentage of the filed area.

### 1.4 Detection of proliferating hepatocyte

At 2 h prior to sacrifice, mice were injected with 5-bromo-2'-deoxyuridine (BrdU, 50mg/kg, i.p.,Sigma). The peroxidase-coupled mouse monoclonal anti-BrdU (DAKO, M0744) and anti-Ki67(DAKO, M7249) were used in subsequent immunohistochemistry study for detecting proliferativehepatocytes. Ten pictures of the interested areas (different portal and central vein areas) per animal were photo-taken under microscopy at x200 magnification. The mean numbers of BrdU-positive orKi67-positive cells of per area per animal were used in statistical analysis.

### 1.5 Western blotting

Tissue lysate were prepared in a buffer containing 50 mM Tris-HCl, pH 7.4, 150 mM NaCl, 0.25%deoxychoic acid, 1% NP40, 1 mM EDTA, 1 mM Na orthovanadate, 1 mM Na fluoride, 1 Mmphenylmethylsulfony fluoride, 1ug/ml aprotinin, 1ug/ml leupeptin and 1 ug/ml peptstain, on ice asdescribed before (Kao CL et al. (2010) Stem Cells Dev 19: 247-2580). The concentrations of sample proteins were determined using the ProteinAssay kit (Bio-Rad, Hercules, CA). Specific amounts of total protein were subjected to 10% SDS-PAGE gel electrophoresis and then transferred to PVDF membranes. Membranes were blocked with 5% non-fat milk and incubated overnight at 4'C with primary antibodies. The membranes were then washed in Tris-buffered saline Tween-20 (TBST) for 5 times and then incubated withhorseradish peroxidase-conjugated secondary antibody for 2 h at room temperature. The membranewas then washed for six times by TBST and specific bands were visualized by ECL (PierceBiotechnology, Rockford, IL) and captured with a digital image system (ChemiGenius2 photodocumentationsystem, Syngenes, Cambridge, UK).

### 1.6 Cytokine array and IP-10 ELISA

The liver tissues were homogenized and prepared in PBS with protease inhibitors (proteaseinhibitors (10 µg/mL Aprotinin, 10µg/mL Leupeptin, and 10 µg/mL Pepstatin) and 1% Triton X-100. The tissue lysates were centrifuged at 10,000 g for 5 minutes to remove cellular debris. Theprotein concentrations were quantified (DC-Bradford protein assay, Bradford, Bio-Rad, Hercules,CA, USA) and 200 µg of proteins were used for the analysis of cytokines by the commercializedassay kits (Mouse cytokine array panel A and IP-10 Immunoassay, R&D, MN) according themanufacture's instruction. The expression of individual cytokines in cytokine array study wasquantified by densitometry and expressed as fold change relative to their expressions in the injuredliver without cell treatment.

### 1.7 Statistical analysis

The results are expressed as mean±SEM. Statistical analysis was performed by using anindependent Student t test and one- and two-way ANOVA with Tukey post hoc test whenappropriate. The survival analysis was performed by using logrank test.A p value <0.05 wasconsidered statistically significant.

### 2. Results

### 2.1 IPS cells alleviated liver injury and promoted regeneration

To establish a liver-injury animal model, we injected mice with CCl4 and evaluated the degree ofhepatocyte injury by measuring ALT and AST. As shown in Fig.16, panel A, the CCl4-injured miceshowed peak levels of serum ALT and AST at 24 hours. Infusion of iPS or iHL cells significantlydecreased ALT and AST levels at 24 and 36 h following CCl4 treatment (n=6, P<0.05) (Fig.16,panel A). We next investigated the hepatocyte proliferation, which is a criticalsign of liver regeneration. BrdU incorporation was used to quantify hepatocytes in S phase. Positiveimmunostaining of Ki67 represents the hepatocytes in cell cycle progression. Only fewproliferating hepatocytes were detected by anti-Ki67 antibody and anti-BrdU antibody in all threegroups at 24 h post-injury (data not shown). Differences in the proliferative response becameobvious at 48 h (Fig. 16,panel B). More than two folds of the proliferating hepatocytes were present in the iPS group compared to the controls. In contrast, the numbers of proliferating hepatocytes in the iHLgroup were significantly lower than that in the iPS group, indicating that only iPS cells have potential topromote liver regeneration.

### 2.2 IPS cells increased hepatic IP-10 expression in injured liver

The iPS cells-induced changes of hepatic cytokines were evaluated by cytokine array (Fig.17,panel A).Among all the cytokines tested, IP-10 and MIG are upregulated by 7- and 6-folds respectively.Further study showed that the mRNA expression of IP-10 and MIG significantly increased at 24 hpost-injury (Fig.17,panel B). In contrast, the expression of iTAC, which belong to the same cytokinefamily as IP-10 and MIG, decreased (Fig.17,panel B). At 48 h post-injury the levels of IP-10 and MIGdecreased, but the expression of IP-10 in the iPS group remainedsignificantly higher than that in the CCl4 group without iPS treatment (p<0.05). At protein levels, results from ELISA and Westernblot analysis demonstrated that a significant increase of hepatic IP-10 by iPS cells at 24 h post-injury(Fig.17,panel C).

### 2.3 IP-10 is an important factor mediated the beneficial effect of iPS cells

From above results, IP-10 could be an important hepatoprotective mediator. We then investigatedwhether or not recombinant IP-10 (rIP-10) promoted the proliferation of injured hepatocytes. Theresults showed that 0.5 or 5ng of rIP-10 sufficiently increased the viability of injured hepatocytes atCCl4 concentration of 1.0 to 2.5 mM (Fig.18,panel A). In injured mice, injection of rIP-10 significantlyreduced the degree of liver damage and the effects of rIP-10 were compatible to iPS cells alone.Combined treatment of rIP-10 and iPS had no additive beneficial effects in injured mice (Fig.18,panel B).The application of anti-IP-10 neutralizing antibody attenuated the protective effects of iPS cells(Fig.18,panel C). In addition, the Ki67 or BrdU staining revealed that the proliferation of hepatocytes atportal regions after iPS infusion was significantly reduced by the anti-IP-10 neutralizing antibody(Fig.18,panel D).

### 2.4 IPS cells and IP-10 improved the survival of repetitive injured mice

To evaluate the survival effects of iPS cells and IP-10, the 72-hour survival rates were evaluated inrepetitive CCl₄-injured mice, to which two additional doses of CCl₄ (given at 24 and 48 hours)were given after the first dose. Half of the repetitive injured mice were randomized into two groupsto receive either iPS cells, or rIP10 (5ng) treatment. Both rIP-10 and IPS groups had significanthigher 72-hour survival rates (100% and 85.7%, respectively) when compared to the untreatedgroup (53.3%, P<0.05) (Fig.18,panel E). No significant difference was noted between iPS and rIP-10 groups.

### References

1. Bouchard, C., Thieke, K., Maier, A., Saffrich, R., Hanley-Hyde, J., Ansorge, W., Reed, S., Sicinski, P., Bartek, J., and M. Eilers. 1999. Direct induction of cyclin D2 by Myc contributes to cell cycle progression and sequestration of p27. EMBO. 18:5321-5333.
2. Chen, L.H., C.C. Loong, T.L. Su, Y.J. Lee, P.M. Chu, M.L. Tsai, P.H. Tsai, P.H. Tu, C.W. Chi, H.C., and S.H. Chiou. 2011. Autophagy inhibition enhances apoptosis triggered by BO-1051, an N-mustard derivative, and involves the ATM signaling pathway. Biochem Pharmacol. 81:594-605.
3. Doege, C.A., K. Inoue, T. Yamashita, D.B. Rhee, S. Travis, R. Fujita, P. Guarnieri, G. Bhagat, W.B. Vanti, A. Shih, R.L. Levine, S. Nik, E.I. Chen, and A. Abeliovich. 2012. Early-stage epigenetic modification during somatic cell reprogramming by Parp1 and Tet2. Nature. 488:652-5.
4. Fan, J., C. Robert, Y.Y. Jang, H. Liu, S. Sharkis, S.B. Baylin, and F.V. Rassool. 2011. Human induced pluripotent cells resemble embryonic stem cells demonstrating enhanced levels of DNA repair and efficacy of nonhomologous end-joining. Mutat Res. 713:8-17.
5. Fujii-Yamamoto, H., J.M. Kim, K. Arai, and H. Masai. 2005. Cell cycle and developmental regulations of replication factors in mouse embryonic stem cells. J Biol Chem. 280:12976-87.
6. Li, H.Y., Y. Chien, Y.J. Chen, S.F. Chen, Y.L. Chang, C.H. Chiang, S.Y. Jeng, C.M. Chang, M.L. Wang, L.K. Chen, S.I. Hung, T.I. Huo, S.D. Lee, and S.H. Chiou. 2011. Reprogramming induced pluripotent stem cells in the absence of c-Myc for differentiation into hepatocyte-like cells. Biomaterials. 32:5994-6005.
7. Liu, M., D.F. Lee, C.T. Chen, C.J. Yen, L.Y. Li, H.J. Lee, C.J. Chang, W.C. Chang, J.M. Hsu, H.P. Kuo, W. Xia, Y. Wei, P.C. Chiu, C.K. Chou, Y. Du, D. Dhar, M. Karin, C.H. Chen, and M.C. Hung. 2012.IKKalpha activation of NOTCH links tumorigenesis via FOXA2 suppression. Mol Cell.45:171-84.
8. Jagtap, P., and C. Szabo. 2005. Poly(ADP-ribose) polymerase and the therapeutic effects of its inhibitors. Nat Rev Drug Discov. 4:421-40.
9. Jin, J., Y.W. Kwon, J.S. Paek, H.J. Cho, J. Yu, J.Y. Lee, I.S. Chu, I.H. Park, Y.B. Park, H.S. Kim, and Y. Kim. 2011. Analysis of differential proteomes of induced pluripotent stem cells by protein-based reprogramming of fibroblasts. J Proteome Res. 10:977-89.
10. Jullien, J., C. Astrand, R.P. Halley-Stott, N. Garrett, and J.B. Gurdon. 2010. Characterization of somatic cell nuclear reprogramming by oocytes in which a linker histone is required for pluripotency gene reactivation. Proc Natl Acad Sci U S A. 107:5483-8.
11. Jullien, J., V. Pasque, R.P. Halley-Stott, K. Miyamoto, and J.B. Gurdon. 2011. Mechanisms of nuclear reprogramming by eggs and oocytes: a deterministic process? Nat Rev Mol Cell Biol. 12:453-9.
12. Kazutoshi Takahashi and Shinya Yamanaka. 2006. Induction of pluripotent Stem Cells from Mouse embryonic and adult fibroblast cultures by defined factors. Cell, 126: 663-676.
13. Kraus, W.L. 2008. Transcriptional control by PARP-1: chromatin modulation, enhancer-binding, coregulation, and insulation. Curr Opin Cell Biol. 20:294-302.
14. Krishnakumar, R., and W.L. Kraus. 2010. The PARP side of the nucleus: molecular actions, physiological outcomes, and clinical targets. Mol Cell. 39:8-24.
15. Maherali, N., R. Sridharan, W. Xie, J. Utikal, S. Eminli, K. Arnold, M. Stadtfeld, R. Yachechko, J. Tchieu, R. Jaenisch, K. Plath, and K. Hochedlinger. 2007. Directly reprogrammed fibroblasts show global epigenetic remodeling and widespread tissue contribution. Cell Stem Cell. 1:55-70.
16. Munoz, J., T.Y. Low, Y.J. Kok, A. Chin, C.K. Frese, V. Ding, A. Choo, and A.J. Heck. 2011. The quantitative proteomes of human-induced pluripotent stem cells and embryonic stem cells. Mol Syst Biol. 7:550.
17. Nakagawa, M., M. Koyanagi, K. Tanabe, K. Takahashi, T. Ichisaka, T. Aoi, K. Okita, Y. Mochiduki, N. Takizawa, and S. Yamanaka. 2008. Generation of induced pluripotent stem cells without Myc from mouse and human fibroblasts. Nat Biotechnol. 26:101-6.
18. Nakagawa, M., N. Takizawa, M. Narita, T. Ichisaka, and S. Yamanaka. 2010. Promotion of direct reprogramming by transformation-deficient Myc. Proc Natl Acad Sci U S A. 107:14152-7.
19. Papp, B., and K. Plath. 2011. Reprogramming to pluripotency: stepwise resetting of the epigenetic landscape. Cell Res. 21:486-501.
20. Phanstiel, D.H., J. Brumbaugh, C.D. Wenger, S. Tian, M.D. Probasco, D.J. Bailey, D.L. Swaney, M.A. Tervo, J.M. Bolin, V. Ruotti, R. Stewart, J.A. Thomson, and J.J. Coon. 2011. Proteomic and phosphoproteomic comparison of human ES and iPS cells. Nat Methods. 8:821-7.
21. Rigbolt, K.T., T.A. Prokhorova, V. Akimov, J. Henningsen, P.T. Johansen, I. Kratchmarova, M. Kassem, M. Mann, J.V. Olsen, and B. Blagoev. 2011 System-wide temporal characterization of the proteome and phosphoproteome of human embryonic stem cell differentiation. Sci Signal. 4:rs3.
22. Sung, L.Y., S. Gao, H. Shen, H. Yu, Y. Song, S.L. Smith, C.C. Chang, K. Inoue, L. Kuo, J. Lian, A. Li, X.C. Tian, D.P. Tuck, S.M. Weissman, X. Yang, and T. Cheng. 2006. Differentiated cells are more efficient than adult stem cells for cloning by somatic cell nuclear transfer. Nat Genet. 38:1323-8.
23. Takahashi, K., K. Tanabe, M. Ohnuki, M. Narita, T. Ichisaka, K. Tomoda, and S. Yamanaka. 2007. Induction of pluripotent stem cells from adult human fibroblasts by defined factors. Cell. 131:861-72.
24. Van Hoof, D., J. Munoz, S.R. Braam, M.W. Pinkse, R. Linding, A.J. Heck, C.L. Mummery, and J. Krijgsveld. 2009. Phosphorylation dynamics during early differentiation of human embryonic stem cells. Cell Stem Cell. 5:214-26.
25. Wang, S., Z. Kou, Z. Jing, Y. Zhang, X. Guo, M. Dong, I. Wilmut, and S. Gao. 2010. Proteome of mouse oocytes at different developmental stages. Proc Natl Acad Sci U S A. 107:17639-44.
26. Yamanaka, S., and H.M. Blau. 2010. Nuclear reprogramming to a pluripotent state by three approaches. Nature. 465:704-12.

## Claims

1. A method for preparing induced pluripotent stem cell (iPSCs) from somatic cells, comprising:
(a) contacting or exposing isolated somatic cells with/to Oct3/4, Sox2, and Parp1; and
(b) culturing the somatic cells as obtained in step (a) under appropriate conditions, thereby converting, at least a subset of, the population of somatic cells into iPSCs and maintaining pluripotency and self-renewal ability.

2. A method for preparing induced pluripotent stem cells (iPSCs) from somatic cells, comprising:
(a) transfecting isolated somatic cells to express Oct3/4, Sox2, and Parp1, wherein the isolated somatic cells are transfected with one or more plasmid or viral vectors comprising Oct3/4, Sox2, and Parp1 operably linked to a promoter; and
(b) culturing the transfected somatic cells as obtained in step (a) under appropriate conditions, thereby converting the somatic cells into iPSCs and maintaining pluripotency and self-renewal ability.

3. The method of claim 1, wherein the method does not comprise a step of transfecting, contacting, or exposing the somatic cells with/to c-Myc, Klf4, Nanog, Lin28, or any combination thereof.

4. The method of claim 2, wherein the method does not comprise a step of transfecting, contacting, or exposing the somatic cells with/to c-Myc, Klf4, Nanog, Lin28, or any combination thereof.

## Patentansprüche

1. Verfahren zur Herstellung von induzierter pluripotenter Stammzelle (iPSCs) aus somatischen Zellen, umfassend:
(a) Inkontaktbringen von isolierten somatischen Zellen mit oder Einwirkenlassen auf diese von Oct3/4, Sox2, und Parp1, und
(b) Kultivieren der somatischen Zellen wie in Schritt (a) erhalten unter geeigneten Bedingungen, damit sich mindestens eine Teilmenge der Population von somatischen Zellen in iPSCs umwandelt und Pluripotenz und Selbsterneuerungsfähigkeit erhalten bleiben.

2. Verfahren zur Herstellung von induzierten pluripotenten Stammzellen (iPSCs) aus somatischen Zellen, umfassend:
(a) Transfizieren von isolierten somatischen Zellen für Expression von Oct3/4, Sox2, und Parp1, wobei die isolierten somatischen Zellen mit einem oder mehr Plasmid- oder viralen Vektoren umfassend Oct3/4, Sox2, und Parp1 funktionsfähig verbunden mit einem Promotor transfiziert werden; und
(b) Kultivieren der transfizierten somatischen Zellen wie in Schritt (a) erhalten unter geeigneten Bedingungen, damit sich die somatischen Zellen in iPSCs umwandeln und Pluripotenz und Selbsterneuerungsfähigkeit erhalten bleiben.

3. Verfahren nach Anspruch 1, wobei das Verfahren keinen Schritt von Transfizieren der somatischen Zellen mit, Inkontaktbringen von diesen mit, oder Einwirkenlassen auf diese von c-Myc, Klf4, Nanog, Lin28, oder jedweder Kombination davon umfasst.

4. Verfahren nach Anspruch 2, wobei das Verfahren keinen Schritt von Transfizieren der somatischen Zellen mit, Inkontaktbringen von diesen mit, oder Einwirkenlassen auf diese von c-Myc, Klf4, Nanog, Lin28, oder jedweder Kombination davon umfasst.

## Revendications

1. Procédé de préparation de cellules souches pluripotentes induites (CSPi) à partir de cellules somatiques, comprenant le fait :
(a) de mettre en contact ou d'exposer des cellules somatiques isolées avec/à Oct3/4, Sox2 et Parp1 ; et
(b) de mettre en culture les cellules somatiques telles qu'obtenues à l'étape (a) dans des conditions appropriées, convertissant ainsi, au moins un sous-ensemble de, la population de cellules somatiques en CSPi et maintenant la pluripotence et la capacité d'autorenouvellement.

2. Procédé de préparation de cellules souches pluripotentes induites (CSPi) à partir de cellules somatiques, comprenant le fait :
(a) de transfecter des cellules somatiques isolées pour exprimer Oct3/4, Sox2 et Parp1, où les cellules somatiques isolées sont transfectées avec un ou plusieurs vecteur(s) plasmidique(s) ou viral/viraux comprenant Oct3/4, Sox2 et Parp1 liés fonctionnellement à un promoteur ; et
(b) de mettre en culture les cellules somatiques transfectées telles qu'obtenues à l'étape (a) dans des conditions appropriées, convertissant ainsi les cellules somatiques en CSPi et maintenant la pluripotence et la capacité d'autorenouvellement.

3. Procédé de la revendication 1, dans lequel le procédé ne comprend pas une étape de transfection, de mise en contact ou d'exposition des cellules somatiques avec/à c-Myc, Klf4, Nanog, Lin28 ou toute combinaison de ceux-ci.

4. Procédé de la revendication 2, dans lequel le procédé ne comprend pas une étape de transfection, de mise en contact ou d'exposition des cellules somatiques avec/à c-Myc, Klf4, Nanog, Lin28 ou toute combinaison de ceux-ci.
